(19) **Europäisches Patentamt / European Patent Office / Office européen des brevets**

(11) **EP 2 014 229 A1**

(12) **EUROPEAN PATENT APPLICATION**
published in accordance with Art. 153(4) EPC

(43) Date of publication:
**14.01.2009 Bulletin 2009/03**

(51) Int Cl.:
**A61B 5/05** (2006.01)

(21) Application number: **07741605.5**

(22) Date of filing: **13.04.2007**

(86) International application number:
**PCT/JP2007/058169**

(87) International publication number:
**WO 2007/125766 (08.11.2007 Gazette 2007/45)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **28.04.2006 JP 2006126484**

(71) Applicant: Omron Healthcare Co., Ltd.
**Kyoto 615-0084 (JP)**

(72) Inventors:
 • **TSENG, Feilang**
  **Kyoto-shi, Kyoto 615-0084 (JP)**
 • **SATO, Tetsuya**
  **Kyoto-shi, Kyoto 615-0084 (JP)**
 • **TOGOE, Yasuyuki**
  **Kyoto-shi, Kyoto 615-0084 (JP)**

(74) Representative: **Wilhelms · Kilian & Partner Patentanwälte**
 **Eduard-Schmid-Strasse 2**
 **81541 München (DE)**

(54) **BODY COMPOSITION DISPLAY SYSTEM CAPABLE OF DISPLAYING MOST SUITABLE BODY COMPOSITION FOR EACH USER**

(57)    A server selects one of a predetermined display specification programs based on at least one of attribute information related to an attribute of the user, purpose information related to a usage purpose of the user, and measurement data of the user input from a communication terminal capable of exchanging data with a body composition measuring instrument (S16). The selected display specification program is transferred to the body composition measuring instrument via the communication terminal (S26, S28). As a result, the display specification program of the body composition measuring instrument is updated (S30).

Fig. 10

EP 2 014 229 A1

**Description**

TECHNICAL FIELD

The present invention relates to a body composition display system, a body composition measuring instrument, a communication terminal, and a server, and in particular to a body composition display system capable of changing the type of body composition displayed as measurement result in the body composition measuring instrument, a body composition measuring instrument, a communication terminal, and a server.

BACKGROUND ART

In a conventional body composition measuring instrument, the type of body composition displayed as measurement result is fixed, such as body fat percentage and muscle percentage irrespective of a usage purpose etc. of a user (e.g., Japanese Laid-Open Patent Publication No. 2005-261488 (hereinafter referred to as Patent Document 1)).

In devices other than the body composition measuring instrument, a device specialized to a specific usage purpose of the user has been proposed. For instance, Japanese Laid-Open Patent Publication No. 2005-52516 (hereinafter referred to as Patent Document 2) discloses a woman body condition management device for measuring an amount of water in a body of a subject, and determining a change in body composition that appears in a monthly period of a woman.

A blood glucose examining instrument capable of upgrading a calculation program of an examination data and a calibration program of the examination data (see Japanese Laid-Open Patent Publication No. 2003-215122 (hereinafter referred to as Patent Document 3)), and a health examination network system capable of downloading software of management terminal (center server) at a patient's terminal and upgrading (see Japanese Laid-Open Patent Publication No. 2002-83066 (hereinafter referred to as Patent Document 4)) are also proposed.

[Patent Document 1] Japanese Laid-Open Patent Publication No. 2005-261488
[Patent Document 2] Japanese Laid-Open Patent Publication No. 2005-52516
[Patent Document 3] Japanese Laid-Open Patent Publication No. 2003-215122
[Patent Document 4] Japanese Laid-Open Patent Publication No. 2002-83066

DISCLOSURE OF THE INVENTION

PROBLEMS TO BE SOLVED BY THE INVENTION

For instance, in households, and the like, one body composition measuring instrument is sometimes used by plural people of different usage purpose (diet to improve lifestyle-related diseases, beautification purposes, etc.) and attribute (age, sex). In such case, the body composition measuring instrument existing conventionally may not be suitable for the usage purpose and the attribute of each user, and drawbacks may occur. The usage purpose might be changed even if one body composition measuring instrument is used by one person, and drawbacks may occur with a single display specification (specification in which the displaying items of the body composition are fixed).

The device of Patent Document 2 is specialized for a specific usage purpose, but as it is for women only, it cannot be used by men, and thus it is not suitable to be used by plural people such as a family. The device capable of upgrading the program exists as in Patent Documents 3 and 4, but a body composition measuring instrument capable of upgrading the program related to the specification of display does not exist.

The present invention has been made to solve the above problems, and an object of the present invention is to provide a body composition display system capable of displaying the body composition of a most suitable type for every user, a body composition measuring instrument, a communication terminal, and a server.

MEANS FOR SOLVING THE PROBLEMS

A body composition display system according to one aspect of the present invention includes a body composition measuring instrument and a server. The body composition measuring instrument includes a measuring section for measuring two or more types of body compositions of a user. The server includes a first storage section for storing a plurality of predetermined display specification programs related to a specification of display of measurement results in the body composition measuring instrument, a selection control section for performing a control to select one of the predetermined display specification programs based on at least one of attribute information related to an attribute of the user, purpose information related to a usage purpose of the user, and measurement data of the user input from a communication terminal capable of exchanging data with the body composition measuring instrument, and a transmitting section for transmitting the display specification program selected by the selection control section to the communication terminal via a communication network as data of the selected program.

Here, "body composition" represents the percentage or the amount of components composing the body (tissues composing the body), and more preferably, represents an index related to the percentage or the amount of components composing the body, or consumption energy and physique of the body. The phrase "two or more types of body compositions" includes at least two components selected from body fat percentage, muscle percentage, fat free mass, amount of body fat, amount of muscle, area of visceral fat, basal metabolism, age index (index representing what average value of what age the basal metabolism corresponds to), and physique index (BMI), and among them, the body fat percentage, the muscle percentage, the area of the visceral fat, and the basal metabolism are preferably included.

Furthermore, "attribute" represents a group classified by at least one of age, sex, or the like. Here, "usage purpose" is a purpose of using the body composition measuring instrument, and includes purposes such as increasing body strength, losing weight to improve lifestyle-related diseases, and losing weight for beautification.

Preferably, the body composition measuring instrument further includes an input/output section for receiving input of the data of the selected program via the communication terminal, a second storage section for storing the input selected program, a display control unit for performing a control to display the measurement result of a specific body composition of the two or more types of body compositions based on the selected program stored in the second storage section, and a display section for displaying according to an output from the display control unit.

Preferably, the communication terminal includes a receiving section for receiving the data of the selected program via the communication network, a second storage section for storing the input selected program, an input/output section for receiving input of the measurement data of the two or more types of body compositions from the body composition measuring instrument, a display control unit for performing a control to display the measurement result of a specific body composition of the two or more types of body compositions based on the selected program stored in the second storage section, and a display unit for displaying according to an output from the display control unit.

Preferably, the first storage section stores each of the predetermined display specification program in correspondence to a plurality of attributes; and the selection control section selects the display specification program corresponded to the attribute of the user indicated by the attribute information when the attribute information is input from the communication terminal.

Preferably, the first storage section stores each of the predetermined display specification program in correspondence to a plurality of usage purposes; and the selection control section selects the display specification program corresponded to the usage purpose of the user indicated by the purpose information when the purpose information is input from the communication terminal.

Preferably, the body composition measuring instrument further includes a third storage section for storing the measurement data of the two or more types of body compositions measured by the measuring section; the input/output section outputs the measurement data stored in the third storage section to the communication terminal; and the selection control section includes an analyzing unit for analyzing the measurement data when the measurement data is input from the communication terminal, and a selecting unit for selecting the display specification program based on the analysis result of the analyzing unit.

Preferably, the communication terminal further includes a third storage section for storing the input measurement data of the two or more types of body compositions; and the selection control section includes an analyzing unit for analyzing the measurement data when the measurement data is input from the communication terminal, and a selecting unit for selecting the display specification program based on the analysis result by the analyzing unit.

A body composition measuring instrument according to another aspect of the present invention includes a measuring section for measuring two or more types of body compositions; a storage section for storing a first display specification program related to specification of display of measurement results by the measuring section; a display control unit for performing a control to display the measurement result of a specific body composition of the two or more types of body compositions based on the first display specification program; a display section for displaying according to an output from the display control unit; an input section for externally receiving input of data of a second display specification program; and an updating section for updating the first display specification program stored in the storage section to the second display specification program.

A communication terminal according to still another aspect of the present invention includes an input/output section for receiving input of measurement data of two or more types of body compositions from a body composition measuring instrument; a storage section for storing a first display specification program related to specification of display of the measurement result; a display control unit for performing a control to display the measurement result of a specific body composition of the two or more types of body compositions based on the first display specification program; a display section for displaying according to an output from the display control unit; a receiving section for externally receiving data of a second display specification program; and an updating section for updating the first display specification program stored in the storage section to the second display specification program.

A server according to yet another aspect of the present invention relates to a server for performing a process of causing a communication terminal capable of exchanging data with a body composition measuring instrument for measuring two or more types of body compositions to display a measurement result in the body composition measuring instrument; the server including a storage section for storing a plurality of predetermined display specification programs related to a specification of display of measurement results in the body composition measuring instrument; a selection control section for performing a control to select one of the predetermined display specification programs based on at least one of attribute information related to an attribute of the user, purpose information related to a usage purpose of the user, and measurement data of the user input from the communication terminal; and a transmitting section for transmitting the display specification program selected by the selection control section to the communication terminal as data of the program for displaying the measurement result of a specific body composition of the two or more types of body compositions on a display section of the communication terminal.

According to the present invention, the display specification program can be freely updated. Therefore, the most suitable type of body composition can be presented for every user.

BRIEF DESCRIPTION OF THE DRAWINGS

Fig. 1 is a diagram showing an overall configuration of a body composition display system according to a first embodiment of the present invention.
Fig. 2 is a view showing one example of an outer appearance of a body composition measuring instrument according to the first embodiment of the present invention.
Fig. 3 is a block diagram showing a hardware configuration of the body composition measuring instrument according to the first embodiment of the present invention.
Fig. 4 is a diagram showing a configuration example of a flash memory in the body composition measuring instrument of the first embodiment of the present invention.
Fig. 5 is a diagram showing an example of a data structure of a measurement result storage region in a flash memory of the body composition measuring instrument of the first embodiment of the present invention.
Fig. 6 is a block diagram showing a hardware configuration of a communication terminal according to the first embodiment of the present invention.
Fig. 7 is a block diagram showing a hardware configuration of a server according to the first embodiment of the present invention.
Fig. 8 is a diagram showing one example of a data structure of a hard disc in the server of the first embodiment of the present invention.
Fig. 9 is a diagram showing a specific example of displaying items of each specification program.
Fig. 10 is a flowchart showing a specification program updating process executed in the body composition display system according to the first embodiment of the present invention.
Fig. 11 is a view showing one example of a screen displayed in step S8 of Fig. 10.
Fig. 12A is a view showing one example of a screen displayed in step S13 of Fig. 10 when attribute is selected for the condition.
Fig. 12B is a view showing one example of a screen displayed in step S13 of Fig. 10 when usage purpose is selected for the condition.
Fig. 13 is a view showing one example of a screen displayed in step S20 of Fig. 10.
Fig. 14 is a view showing one example of a screen displayed on a display section of the body composition measuring instrument when the specification program is updated in step S30 of Fig. 10.
Fig. 15 is a flowchart showing a body composition measuring process executed by the control section of the body composition measuring instrument according to the first embodiment of the present invention.
Fig. 16A is a view showing one example of a screen displayed in step S118 of Fig. 15.
Fig. 16B is a view showing one example of a screen displayed in step S118 of Fig. 15.
Fig. 17 is a flowchart showing a specification program updating process executed in a body composition display system according to a second embodiment of the present invention.
Fig. 18 is a view showing one example of a screen displayed at the communication terminal after the process of step S212 of Fig. 17.
Fig. 19 is a diagram showing a configuration example of a flash memory of a body composition measuring instrument according to a third embodiment of the present invention.
Fig. 20 is a diagram showing a configuration example of a flash memory of a communication terminal of the third embodiment of the present invention.
Fig. 21 is a flowchart showing a measurement result storing process executed by the body composition measuring instrument and the communication terminal according to the third embodiment of the present invention.

Fig. 22 is a flowchart of a sub-routine showing a body composition measuring process according to the third embodiment of the present invention.

Fig. 23 is a flowchart showing a specification program updating process executed by the communication terminal and the server according to the third embodiment of the present invention.

Fig. 24 is a flowchart showing a measurement result displaying process executed by the communication terminal according to the third embodiment of the present invention.

Fig. 25 is a view showing one example of a screen displayed in step S608 of Fig. 24.

DESCRIPTION OF SYMBOLS

1: upper limb unit, 2: lower limb unit, 3: cable, 10b, 10c: grip, 10a: main body, 11: detecting section, 12: control section, 13: timer, 14: memory, 15, 205: display section, 16, 204: operating section, 17: power supply section, 18: data input/output section, 20: accommodating section, 22: weight measuring section, 100: body composition measuring instrument, 121: body composition calculating unit, 122: display control unit, 141, 202, 302: ROM, 142, 203, 303: RAM, 143, 206: flash memory, 200: communication terminal, 201, 301: CPU, 207, 307: communication I/F, 209: input/output I/F, 208: drive device, 300: server, 306: hard disc, 400: communication network, 410: recording medium, 1000: body composition display system, E10: hands electrode, E20: feet electrode, E11, E12, E13, E14, E21, E22, E23, E24: electrode.

BEST MODE FOR CARRYING OUT THE INVENTION

Embodiments of the present invention will be described in detail with reference to the drawings. Same reference numerals are denoted for the same or corresponding portions throughout the drawings.

[First embodiment]

<Configuration of body composition display system according to first embodiment of the present invention>

(Regarding overall configuration of body composition display system)

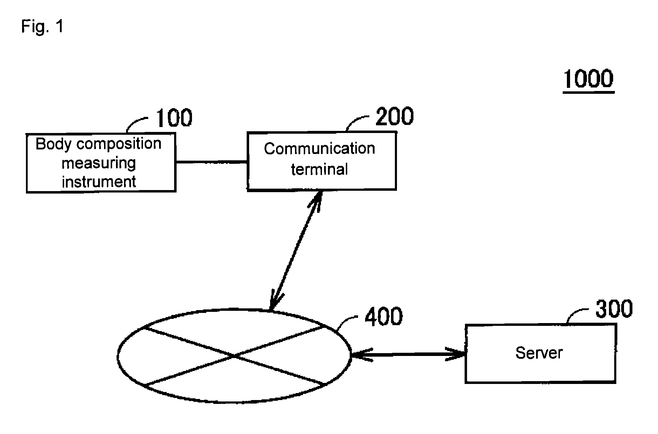

Fig. 1 is a diagram showing an overall configuration of a body composition display system 1000 according to a first embodiment of the present invention.

With reference to Fig. 1, the body composition display system 1000 includes a body composition measuring instrument 100, a communication terminal 200 capable of exchanging data with the body composition measuring instrument 100, and a server connected to the communication terminal 200 by way of a communication network 400. The body composition measuring instrument 100 and the communication terminal 200 may be wired connected or may be wireless connected.

(Outer appearance and configuration of body composition measuring instrument)

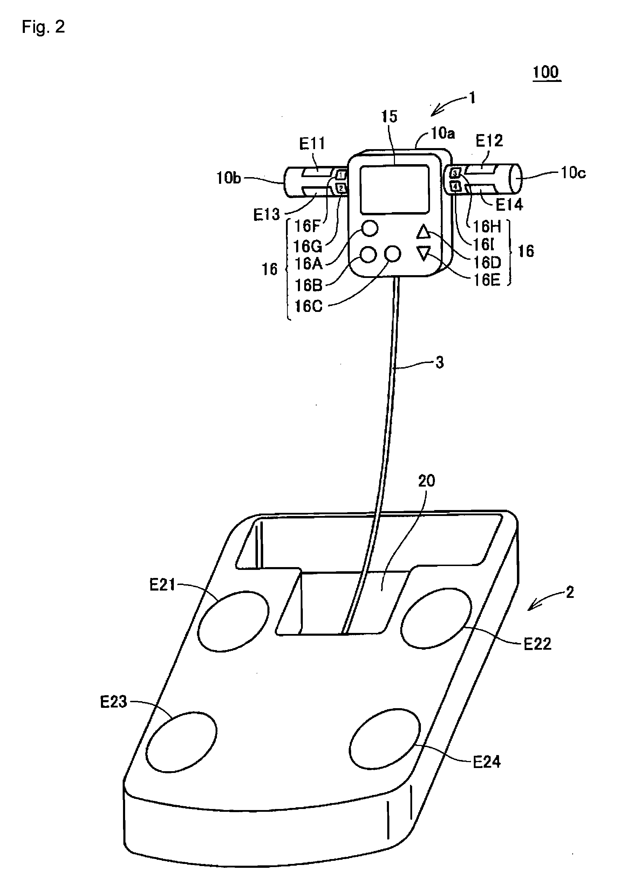

Specific examples of the outer appearance and the configuration of the body composition measuring instrument 100 according to the first embodiment of the present invention will be described using Figs. 2 to 5.

Fig. 2 is a view showing one example of the outer appearance of the body composition measuring instrument 100 according to the first embodiment of the present invention.

With reference to Fig. 2, the body composition measuring instrument 100 includes an upper limb unit 1 that can be gripped by the user with both hands, a lower limb unit 2 on which both feet of the user can be placed, and a cable 3 for electrically connecting the upper limb unit 1 and the lower limb unit 2.

The upper limb unit 1 includes a body 10a and grips 10b, 10c arranged on the left and the right of the body 10a. The body 10a is provided with a display section 15 for displaying measurement results and various information, and an operating section 16 operated by the user to accept instructions and input of various information from the user. A plurality of electrodes E11, E12, E13, and E14 is provided on the grips 10b, 10c. The grips 10b, 10c are configured to be able to grip by the user with both hands. The electrodes E11, E13 are provided on the grip 10b for the left hand, and the electrodes E12, E14 are provided on the grip 10c for the right hand. The electrodes E11, E12 provided on the upper side (head side of the user in the measuring pose) of the respective grips 10b, 10c are current application electrodes, and the electrodes E13, E14 provided on the lower side of the respective grips 10b, 10c are voltage detection electrodes. Here, description is made such that the upper limb unit 1 includes the grips 10b, 10c configured in a handle shape, but is not limited thereto. The user merely needs to be able to grip the upper limb unit 1 with both hands and the electrodes E11 to E14 merely need to be provided at the portion to be gripped with both hands. That is, the electrodes E11, E13 merely need to contact the left hand of the user and the electrodes E12, E14 merely need to contact the right hand.

A plurality of electrodes E21, E22, E23, and E24 are provided on the upper surface (surface on which the user places both feet) of the lower limb unit 2. Among such electrodes, the electrodes E21, E22 provided on the front side (toe side of the user in the measuring pose) of the lower limb unit 2 are current application electrodes, and the electrodes E23, E24 provided on the back side (heel side of the user in the measuring pose) of the lower limb unit 2 are voltage detection electrodes. The lower limb unit 2 includes an accommodating section 20 for accommodating the upper limb unit 1.

In the following description, the electrodes E11 to E14 are collectively referred to as "hand electrodes E10" and the electrodes E21 to E24 are collectively referred to as "feet electrodes E20".

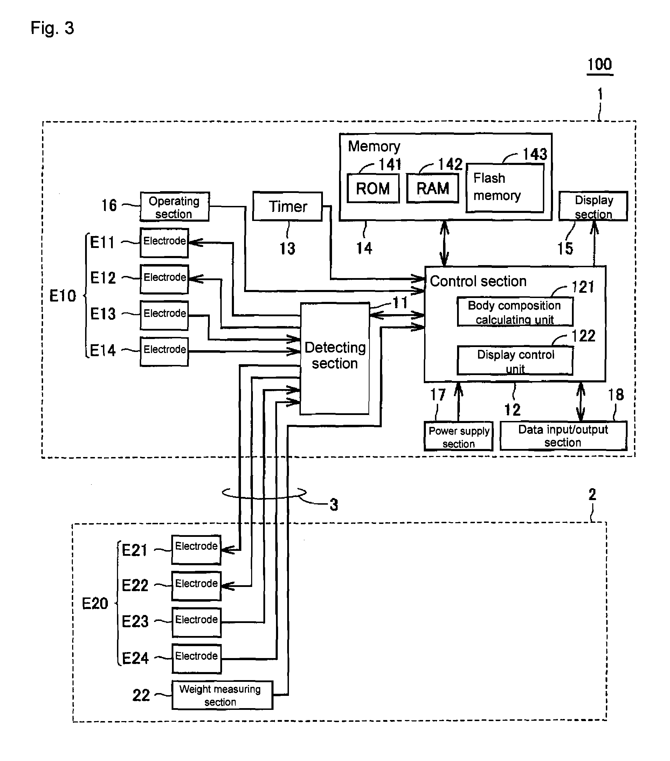

Fig. 3 is a block diagram showing a hardware configuration of the body composition measuring instrument 100 according to the first embodiment of the present invention.

In addition to the hand electrodes E10, the display section 15, and the operating section 16 described above, the upper limb unit 1 further includes a detecting section 11 for detecting the potential difference between the hand and the foot (whole body) by applying current between the hands and the feet of the user with both the hand electrodes E10 and the feet electrodes E20; a control section 12 for controlling the entire body composition measuring instrument 100; a timer 13 for measuring date and time; a memory 14 for storing various data and programs; a power supply section 17 for supplying power to the control section 12; and a data input/output section 18 for exchanging data with the communication terminal 200.

The lower limb unit 2 desirably further includes a weight measuring section 22 for measuring the weight of the user, in addition to the feet electrodes E20 described above. The weight measuring section 22 is configured by a sensor, and the like.

The memory 14 is configured by a ROM 141 for storing programs and data in advance, a RAM 142 for temporarily recording work data, and a non-volatile memory such as a flash memory 143. The configuration example of the flash memory 143 will be described hereinafter in detail.

The display section 15 is configured by an LCD (Liquid Crystal Display) and the like. The operating section 16 includes a plurality of buttons, and the like. As shown in Fig. 2, the operating section 16 includes a power button 16A for instructing ON/OFF of the power supply, a memory button 16B for instructing display of past measurement information, a measurement button 16C for instructing start of measurement, and up and down buttons 16D, 16E for moving the cursor (not shown) displayed on the display section 15 to upward, downward, to the right, and to the left, or increasing or decreasing the numerical values in the display. The operating section 16 may include a plurality of, for example, four personal number buttons 16F, 16G, 16H, and 16I so that a plurality of users can use the body composition measuring instrument 100. In the present embodiment, description is made on the assumption that the personal number buttons 16F, 16G, 16H, 16I are included in the operating section 16.

The detecting section 11 is controlled by the control section 12 to switch the electrodes. The detecting section 11 preferably detects the potential difference between the hands or the feet by applying current to the hands or the feet of the user by either one of the hand electrodes E10 or the feet electrodes E20. The information on the detected potential difference is output to the control section 12. The detecting section 11 includes a switching switch (not shown), connected to all of the hand electrodes E10 and the feet electrodes E20, for switching the electrodes in response to the instruction from the control section 12, and a constant current generating unit (not shown) for flowing a constant current to at least one pair of current electrodes selected by the switching switch, wherein the potential difference of at least one pair of voltage electrodes selected by the switching switch is detected with a constant current being applied to the user through the current electrodes.

The control section 12 is configured by a CPU (Central Processing Unit) and the like. The control section 12 includes a body composition calculating unit 121 for calculating two or more types of body composition of the user based on the program previously stored in the ROM 141, and a display control unit 122 for performing a control to display the calculation result by the body composition calculating unit 121 on the display section 15 based on the specification program to be hereinafter described in detail.

The body composition calculating unit 121 measures a whole body impedance, a hands impedance and a feet impedance respectively based on the potential difference between the hands and the feet, both hands, and both feet detected by the detecting section 11. Various body compositions of the user are calculated based on such measured impedances.

Each of impedance is specifically measured as follows. When measuring the whole body impedance, the body composition calculating unit 121 flows current from the electrodes E11, E12 to the electrodes E21, E22, and performs a control to detect the potential difference between the electrodes E13, E14 and the electrodes E23, E24 with current being applied to the whole body of the user. The whole body impedance is calculated (measured) based on the whole body potential difference detected in this manner. When measuring the whole body impedance, the electrode E11 and the electrode E12, the electrode E21 and the electrode E22, the electrode E13 and the electrode E14, as well as the electrode E23 and the electrode E24 are preferably short-circuited. When measuring the hands impedance, the body composition calculating unit 121 flows current between the electrode E11 and the electrode E12, and performs a control to detect the potential difference between the electrode E13 and the electrode E14 with current being applied between both hands of the user. When measuring the feet impedance, the body composition calculating unit 121 flows current between the electrode E21 and the electrode E22, and performs a control to detect the potential difference between the electrode E23 and the electrode E24 with current being applied between both feet of the user.

In the present embodiment, the body composition calculating unit 121 calculates four types of body composition such as body fat percentage, muscle percentage, visceral fat area (hereinafter simply referred to as "visceral fat"), and basal metabolism based on the whole body impedance, the hands impedance, and the feet impedance.

The calculation formula of the body fat percentage (%FAT) is expressed with the following equations (1) and (2).

$$\%FAT = (W-FFM)/W \cdot 100 \qquad \ldots (1)$$

$$FFM = a \cdot H^2/Zw + b \cdot W + c \cdot Ag + d \qquad \ldots (2)$$

(wherein, FFM: fat free mass, W: weight, H: height, Zw: whole body impedance, Ag: age, a to d: constant) The constants a to d are predefined by correlation with a reference measured with DEXA (Dual Energy X-ray Absorptiometry) and the like. The constants a to d may differ according to sex.

The method of calculating the body fat percentage is not particularly limited, and may be calculated through known methods. The muscle percentage, the visceral fat area, and the basal metabolic rate may also be calculated through known methods.

In the present embodiment, the display control unit 122 performs a control to display the calculation result of the specific body composition of the four types of body compositions calculated by the body composition calculating unit 121 on the display section 15. A specific process by the display control unit 122 will be hereinafter described.

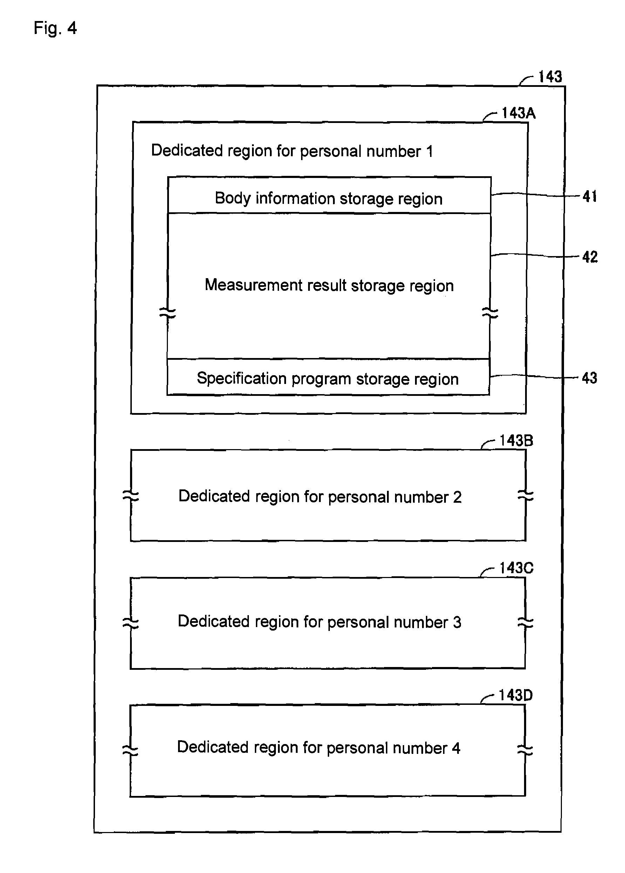

A configuration example of the flash memory 143 will be described in detail below. Fig. 4 is a diagram showing a configuration example of the flash memory 143 of the body composition measuring instrument 100 according to the first embodiment of the present invention.

With reference to Fig. 4, the flash memory 143 includes a region 143A for storing information about the user corresponding to a personal number 1, a region 143B for storing information about the user corresponding to a personal number 2, a region 143C for storing information about the user corresponding to a personal number 3, and a region 143D for storing information about the user corresponding to a personal number 4.

The region 143A includes a body information storage region 41 for storing body information of the user corresponding to the personal number 1, a measurement result storage region 42 for storing the measurement result of the user corresponding to the personal number 1, and a specification program storage region 43 for storing the specification program of the user corresponding to the personal number 1. The regions 143B to 143D corresponding to other personal numbers also include storage regions similar to those of the region 143A.

The "specification program" is a program related to specification of display of the measurement result in the body composition measuring instrument 100, and for example, is a program defined with which body composition of the four types of body compositions is to be displayed.

The "body information" is information necessary for calculating the body composition, and includes, age, sex, height, weight, and the like. The body composition measuring instrument 100 according to the present embodiment includes the weight measuring section 22, and thus age data, sex data, and height data based on the input from the user of the body information are stored in the body information storage region 41.

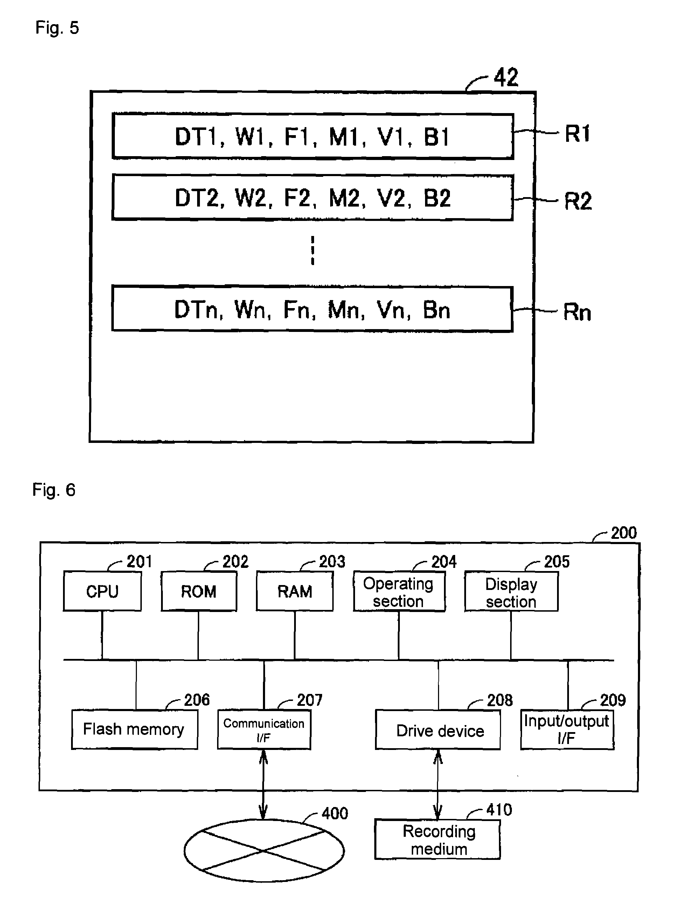

One example of a data structure of the measurement result storage region 42 is shown in Fig. 5. When a body composition measuring process described in detail hereinafter is executed, the measurement result is stored in units of records R in the measurement result storage region 42 corresponding to the personal number specified by the user. The record R (R1, R2, ..., Rn) includes date and time data DT in body composition measurement (e.g., in potential difference detection), weight value data W, body fat percentage data F, muscle percentage data M, visceral fat data V, and basal metabolic mass data B. Such data merely need to be stored in each region in correspondence for every measurement, and it is not limited to the storage form using the record R.

(Regarding configuration of communication terminal)

With reference to Fig. 6, one example of a configuration of the communication terminal 200 according to the first embodiment of the present invention will be described. Fig. 6 is a block diagram showing a hardware configuration of the communication terminal 200 according to the first embodiment of the present invention. The communication terminal 200 is assumed to be a portable telephone. However, it is not limited to portable telephones, and may be a PC (Personal Computer), a PDA (Personal Digital Assistant), and the like.

With reference to Fig. 6, the communication terminal 200 includes a CPU 201 for controlling the entire communication terminal 200, a ROM 202 storing programs and data in advance, a RAM 203 for temporarily recording work data, an operating section 204 for receiving instructions and input of various information from the user, a display section 205 for displaying information, a non-volatile memory such as a flash memory 206, a communication I/F 207 connected with the communication network 400, a drive device 208 for performing write and read of data with respect to a recording medium 410, and an input/output I/F 209 for exchanging data with the body composition measuring instrument 100.

(Regarding configuration of server)

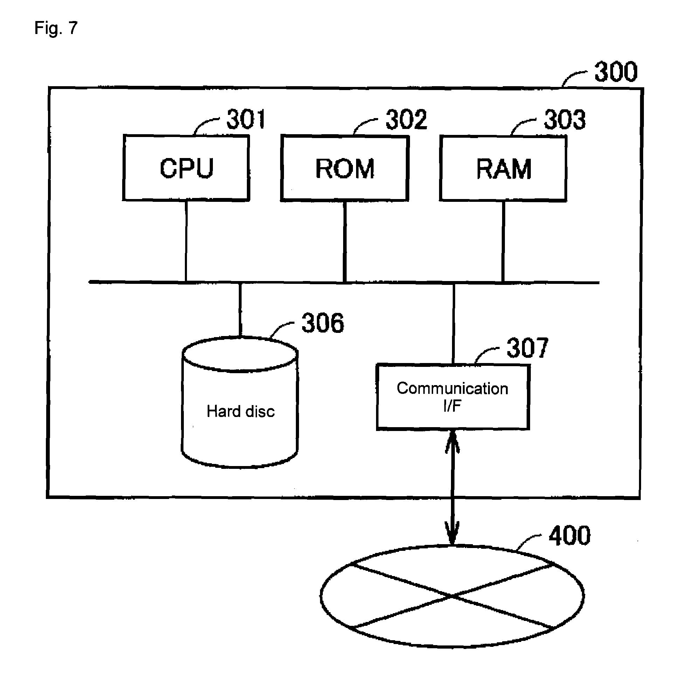

One example of a configuration of the server 300 according to the first embodiment of the present invention will be described using Figs. 7 to 9.

Fig. 7 is a block diagram showing a hardware configuration of the server 300 according to the first embodiment of the present invention.

With reference to Fig. 7, the server 300 includes a CPU 301 for controlling the entire server 300, a ROM 302 storing programs and data in advance, a RAM 303 for temporarily recording the work data, a hard disc 306, and a communication I/F 307 connected with the communication network 400.

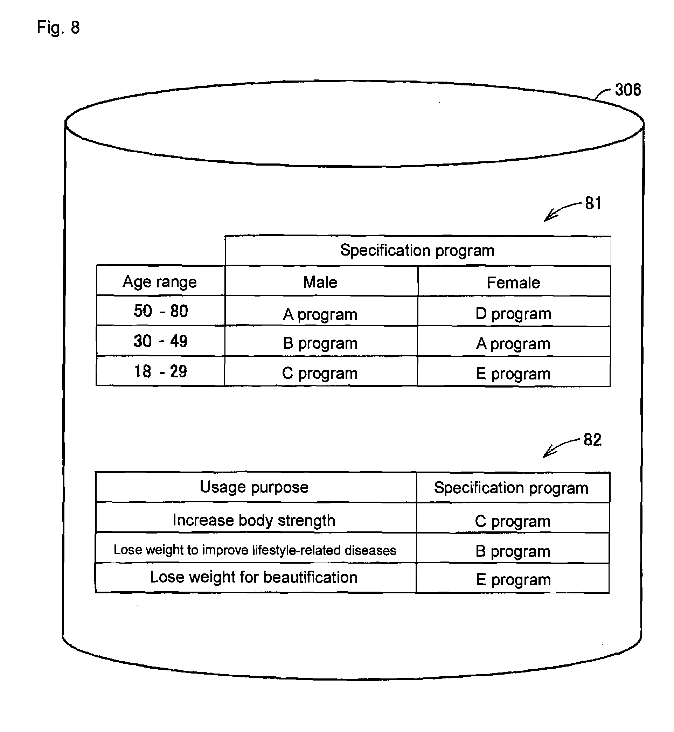

Fig. 8 is a diagram showing one example a data structure of the hard disc 306 in the server 300.

With reference to Fig. 8, the hard disc 306 includes an attribute correspondence table 81 and a usage purpose correspondence table 82 in advance. In the attribute correspondence table 81, each of a plurality of predetermined specification programs is stored in correspondence to a plurality of attributes. In the present embodiment, "attribute" represents a group classified by age and sex. For instance, a male between an age of 50 and 80 is corresponded to an A program, and a female between an age of 50 and 80 is corresponded to a D program. A male between an age of 30 and 49 is corresponded to a B program, and a female between an age of 30 and 49 is corresponded to an A program. A male between an age of 18 and 29 is corresponded to a C program, and a female between an age of 18 and 29 is corresponded to an E program.

In the usage purpose correspondence table 82, each of a plurality of predetermined specification programs is stored in correspondence to a plurality of usage purposes. In the present embodiment, the "usage purpose" includes purposes of increasing body strength, losing weight to improve lifestyle-related diseases, and losing weight for beautification. For instance, if the usage purpose is "increase body strength", the C program is corresponded, and if the usage purpose is "losing weight to improve lifestyle-related diseases", the B program is corresponded. If the usage purpose is "losing weight for beautification", the E program is corresponded.

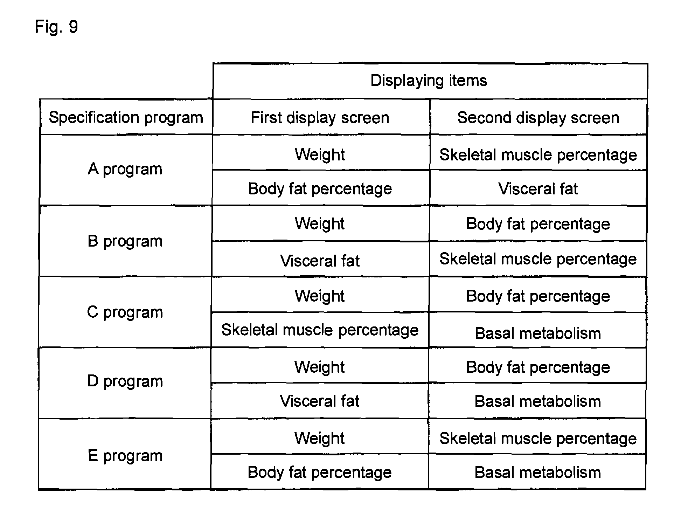

Fig. 9 is a diagram showing a specific example of the displaying items of each specification program. In the present embodiment, the weight is displayed irrespective of the type of the specification program, and the four types of body compositions and the weight are collectively referred to as "body feature index".

In Fig. 9, each specification program specifically shows which type (item) of the body feature indexes (four types of body composition, weight), and also shows the specific example of the priority of the displaying item (order and/or position of the displaying item).

When the specification program is the A program, the displaying items of the first display screen are the weight and the body fat percentage, and the displaying items of the second display screen are the skeletal muscle percentage and the visceral fat. When the specification program is the B program, the displaying items of the first display screen are the weight and the visceral fat, and the displaying items of the second display screen are the body fat percentage and the skeletal muscle percentage. When the specification program is the C program, the displaying items of the first display screen are the weight and the skeletal muscle percentage, and the displaying items of the second display screen are the body fat percentage and the basal metabolism. When the specification program is the D program, the displaying items of the first display screen are the weight and the visceral fat, and the displaying items of the second display screen are the body fat percentage and the basal metabolism. When the specification program is the E program, the displaying items of the first display screen are the weight and the body fat percentage, and the displaying items of the second display screen are the skeletal muscle percentage and the basal metabolism.

<Operation of the body composition display system and the body composition measuring instrument according to the first embodiment of the present invention>

(Regarding specification program updating process)

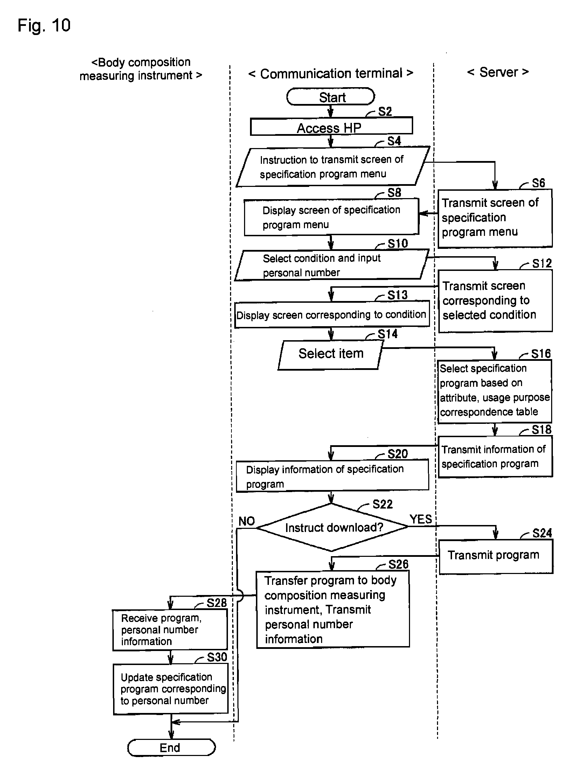

The specification program updating process executed by the body composition display system 1000 according to the first embodiment of the present invention will be described using Figs. 10 to 14.

Fig. 10 is a flowchart showing the specification program updating process executed by the body composition display system 1000 according to the first embodiment of the present invention.

With reference to Fig. 10, the communication terminal 200 accesses a home page provided by the server 300 based on instruction from the user (step S2). When the transmission instruction of a menu screen of the specification program is input by the user (step S4), the server 300 transmits the menu screen of the specification program (step S6).

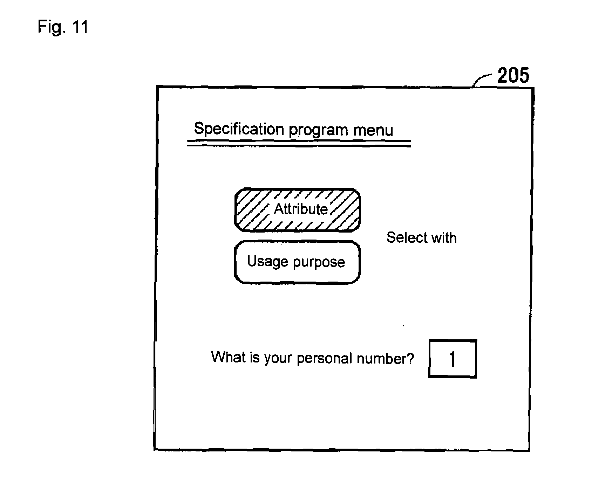

The communication terminal 200 displays the transmitted menu screen of the specification program on the display section 205 (step S8). One example of a screen displayed in step S8 is shown in Fig. 11.

With reference to Fig. 11, the display section 205 is displayed with items (buttons) respectively indicating the two conditions (attribute, usage purpose) of when selecting the specification program, and an input field for inputting the personal number of the user.

When the selection of the condition and the input of the personal number are made by the user with such screen displayed on the display section 205 (step S10), the server 300 transmits a screen corresponding to the selected condition (step S12). In Fig. 11, the attribute is selected as the selecting condition of the specification program, and 1 is input as the personal number. The data of the personal number input in step S10 is temporarily recorded in the RAM 203.

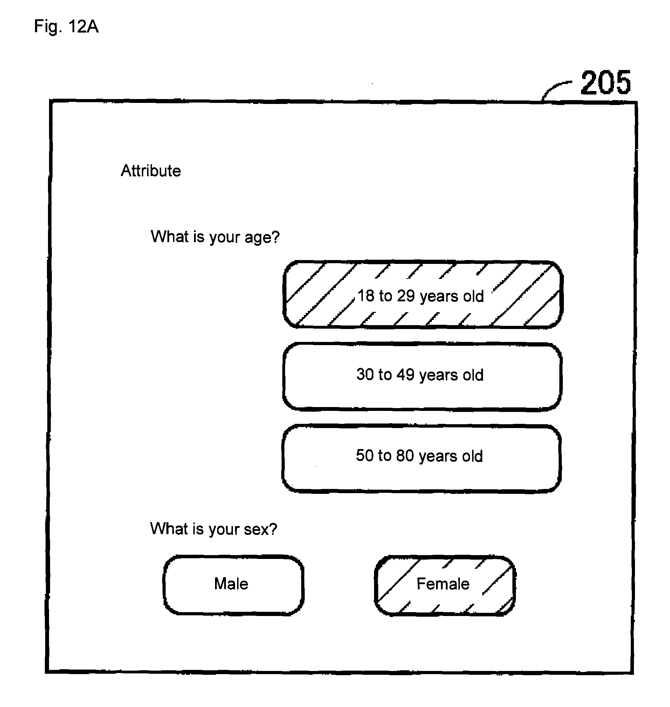

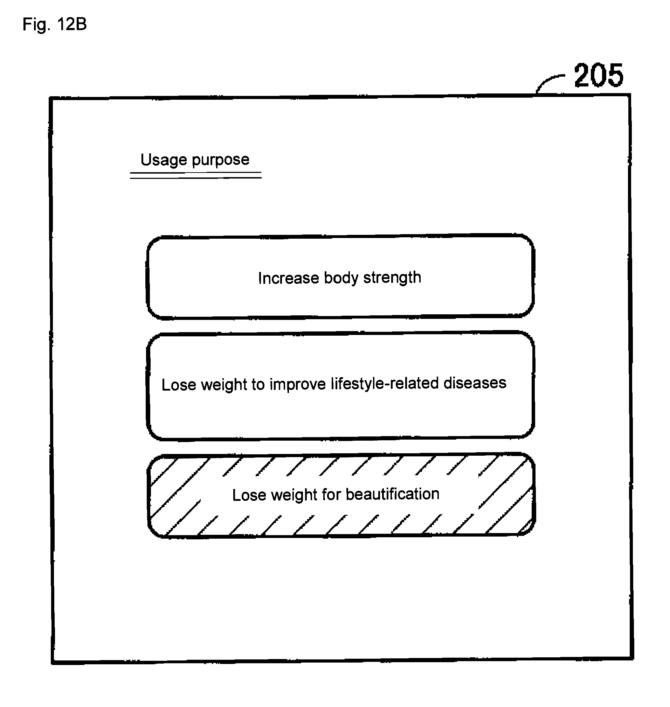

When the screen corresponding to the selected condition is transmitted by the server 300, a screen corresponding to the selected condition is displayed on the display section 205 of the communication terminal 200 (step S13). One example of the screen displayed in step S13 is shown in Fig. 12.

Fig. 12A is one example of a screen displayed when attribute is selected as the condition, and Fig. 12B is one example of a screen displayed when usage purpose is selected as the condition. With reference to Fig. 12A, a plurality of items (item for selecting 18 to 29 years old, item for selecting 30 to 49 years old, and item for selecting 50 to 80 years old) for accepting the input of the age range of the user, and two items for accepting the input of sex of the user are displayed on the display section 205. With reference to Fig. 12B, items corresponding to the three usage purposes are displayed on the display section 205.

When the user selects the items with the screen shown in Fig. 12A or Fig. 12B being displayed (step S14), the CPU 301 of the server 300 selects the specification program based on the attribute correspondence table 81 or the usage purpose correspondence table 82 (step S16). For instance, when the user specifies attribute as the selected condition of the specification program, and selects female in the age range of 18 to 29 years old as the attribute, the CPU 301 selects the E program with reference to the attribute correspondence table 81.

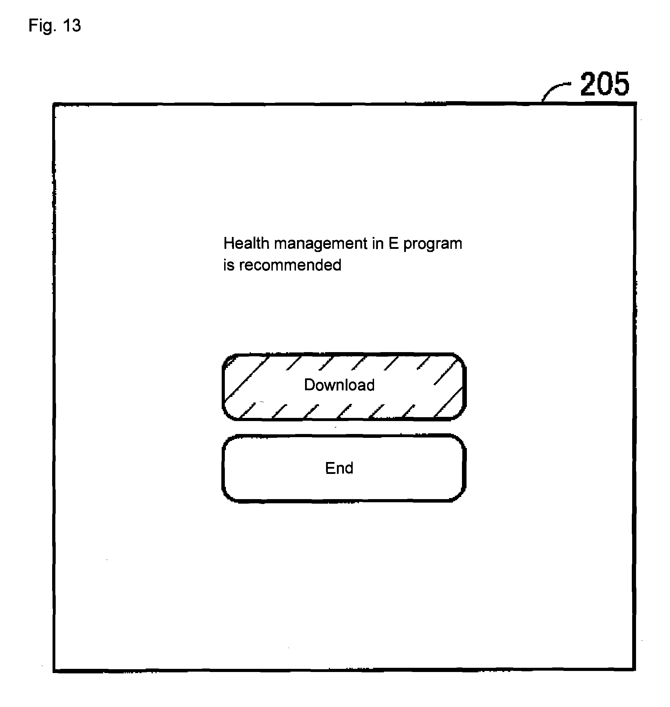

Subsequently, the server 300 transmits information of the specification program selected in step S16 to the communication terminal 200 (step S18). The information of the specification program transmitted by the server 300 is then displayed on the display section 205 of the communication terminal 200 (step S20). One example of the screen displayed in step S20 is shown in Fig. 13.

With reference to Fig. 13, a message such as "Health management in E program is recommended" and items for selecting download and end are displayed on the display section 205.

When the user inputs instruction of download with the screen shown in Fig. 13 being displayed (YES in step S22), the server 300 transmits the data of the specification program selected in step S16 to the communication terminal 200 via the communication I/F 307 (step S24). The communication terminal 200 receives the program transmitted by the server 300 via the communication I/F 207, and transfers the same to the body composition measuring instrument 100 via the input/output I/F 209 (step S26). In this case, the communication terminal 200 also transmits information of the personal number input in step S10 to the body composition measuring instrument 100.

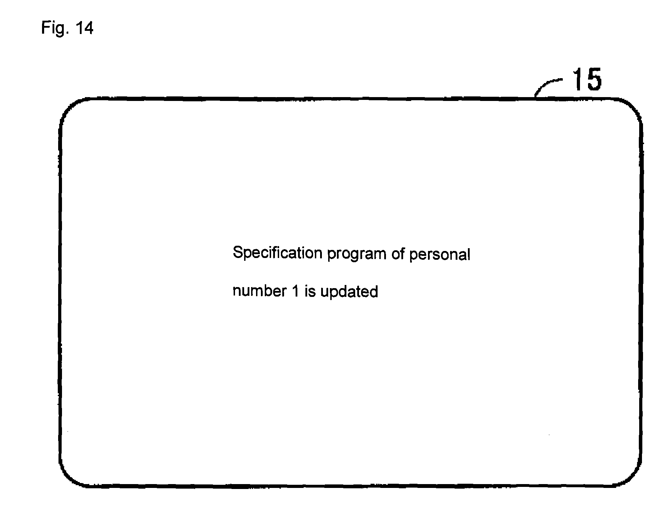

The data input/output section 18 of the body composition measuring instrument 100 receives the data of the specification program and the information of the personal number (step S28). The control section 12 of the body composition measuring instrument 100 then updates the specification program corresponding to the relevant personal number (step S30). More specifically, the control section 12 updates the specification program stored in the specification program storage region 43 of the regions 143A to 143D corresponding to the relevant personal number to the received specification program. After the updating of the specification program is terminated, the control section 12 may inform the user that the specification program has been updated. Specifically, for example, a screen shown in Fig. 14 is displayed on the display section 15. With reference to Fig. 14, a message "specification program of personal number 1 is updated" is displayed on the display section 15.

When instruction to download is not input by the user in step S22, that is, if end is selected (NO in step S22), the specification program updating process is terminated without updating the specification program.

(Regarding body composition measuring process)

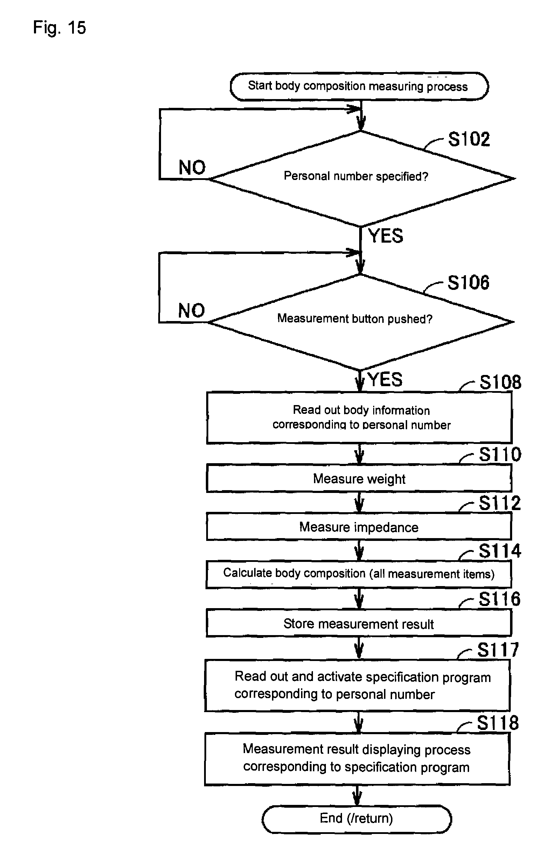

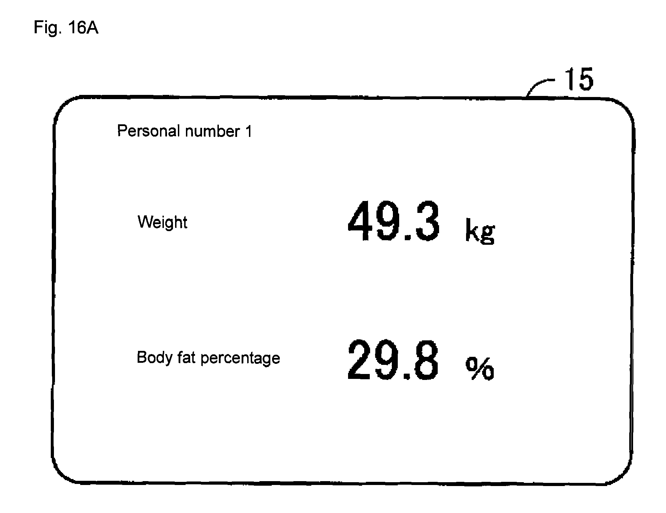

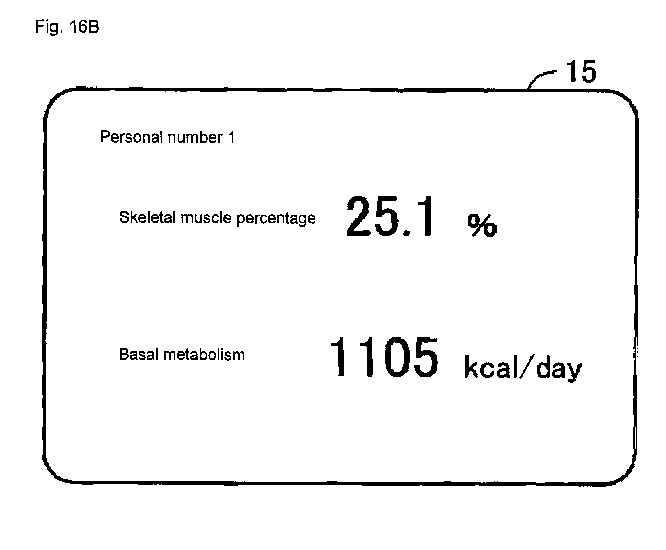

The body composition measuring process executed by the body composition measuring instrument 100 according to the first embodiment of the present invention will be described using Figs. 15 and 16.

Fig. 15 is a flowchart showing the body composition measuring process executed by the control section 12 of the body composition measuring instrument 100 according to the first embodiment of the present invention.

With reference to Fig. 15, the control section 12 first determines whether or not the personal number is specified by the user (step S102). That is, whether or not one of the buttons 16F to 16I is pushed by the user is determined. The control section 12 waits until the personal number is specified (NO in step S102). When determined that the personal number is specified (YES in step S102), the process proceeds to step S106.

In step S106, the control section 12 determines whether or not the measurement button 16C is pushed, and waits until the measurement button 16C is pushed (NO in step S106). When the measurement button 16C is pushed (YES in step S106), the process proceeds to step S108.

In step S108, the body composition calculating unit 121 reads out body information (height, age, sex) corresponding to the personal number specified by the user. For instance, it is assumed that the personal number switch 16F corresponding to personal number 1 is pushed in step S102. In this case, the height data, the age data, and the sex data are read out from the body information storage region 41 in the region 143A in step S108. The read body information are temporarily recorded in an internal memory.

The body composition calculating unit 121 then measures the weight based on the signal from the weight measuring section 22 (step S110). The measured weight value is temporarily recorded in the internal memory.

The body composition calculating unit 121 then executes an impedance measurement process (step S112). Specifically, the detecting section 11 is controlled, and the whole body impedance, the hands impedance, and the feet impedance are measured. The value of the respective measured impedance is temporarily recorded in the internal memory.

The body composition calculating unit 121 calculates four types of body compositions of the user based on each data temporarily recorded in the internal memory, the predetermined calculation formula described above, and the like (step S114). The control section 12 then stores the measurement result, that is, the value of the body composition calculated in step S114 in the measurement result storage region 42 in the region 143A (step S116).

The control section 12 reads out the specification program corresponding to the personal number specified in step S102 and activates the same (step S117). In shipping, a predetermined specification program (e.g., display only weight and body fat percentage) may be stored in each specification program storage region 43. Alternatively, in shipping, a predetermined specification program may be stored only in the ROM 141 and not in each specification program storage region 43. In this case, if the specification program is not stored in the specification program storage region 43 corresponding to the personal number in step S117, the control section 12 reads out the predetermined specification program from the ROM 141 and activates the relevant program.

When the specification program is activated, the display control unit 122 executes the measurement result displaying process corresponding to the specification program (step S118). That is, the display control unit 122 displays the measurement results of the weight and the specific body composition of the measurement data for this time according to the specification program. The measuring process is then terminated.

Fig. 16 is a view showing one example of a screen displayed in step S118 of Fig. 15.

In Fig. 16, an example of when the specification program of the user specified with personal number 1 is the E program is shown. In step S118, the weight and the body fat percentage are first displayed on the display section 15 as shown in Fig. 16A. Thereafter when a predetermined time has elapsed, the skeletal muscle percentage and the basal metabolism are displayed as shown in Fig. 16B. Thus, even with one body composition measuring instrument, the body composition (body feature index) of the type suited for the attribute or the usage purpose of the user are displayed. As shown in Fig. 16A or Fig. 16B, the screen display does not need to change with elapse of time, and the four body feature indexes may be simultaneously displayed, such as information shown in Fig. 16A may be displayed on the upper level and the information shown in Fig. 16B may be displayed on the lower level.

As described above, according to the present embodiment, each user of the body composition measuring instrument 100 can freely update the specification program. Thus, the measurement result corresponding to the attribute or the usage purpose of each user can be displayed with one body composition measuring instrument 100. Furthermore, even if the usage purpose of the user is changed, it can be responded with one body composition measuring instrument 100.

In the present embodiment, the user can select either one of the attribute or the usage purpose as the selecting condition of the specification program in the specification program updating process, but they may not be selectable. In this case, only one of the attribute correspondence table 81 and the usage purpose correspondence table 82 may be stored in the hard disc 306 of the server 300.

In the present embodiment, only the displaying items are differed according to the specification program, but the measurement items may also be differed according to the specification program. That is, only the measurement of the body composition corresponding to the displaying item may be executed in the body composition measuring instrument 100. In this case, the body composition calculating unit 121 calculates the body composition (same as displaying item) of the item based on the specification program.

In the present embodiment, the specification program of the body composition measuring instrument 100 is updated by accessing the home page provided by the server 300 with the communication terminal 200, and downloading the specification program. However, the mode is not limited thereto, and the specification program may be updated by installing the specification program to the body composition measuring instrument 100 from a recording medium recorded with a plurality of specification programs (A program to E program) in advance. In this case, the data input/output section 18 can read out data recorded on the recording medium (not shown) such as CD-ROM (Compact Disc-ROM).

[Second embodiment]

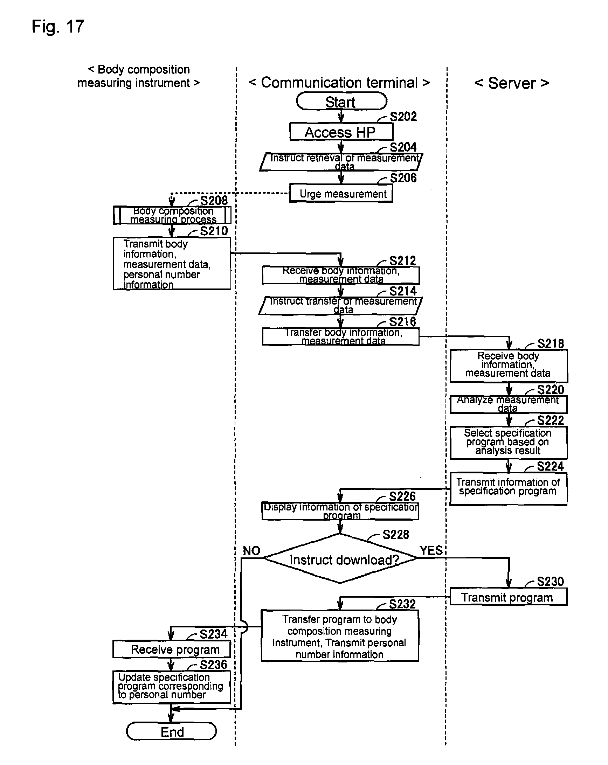

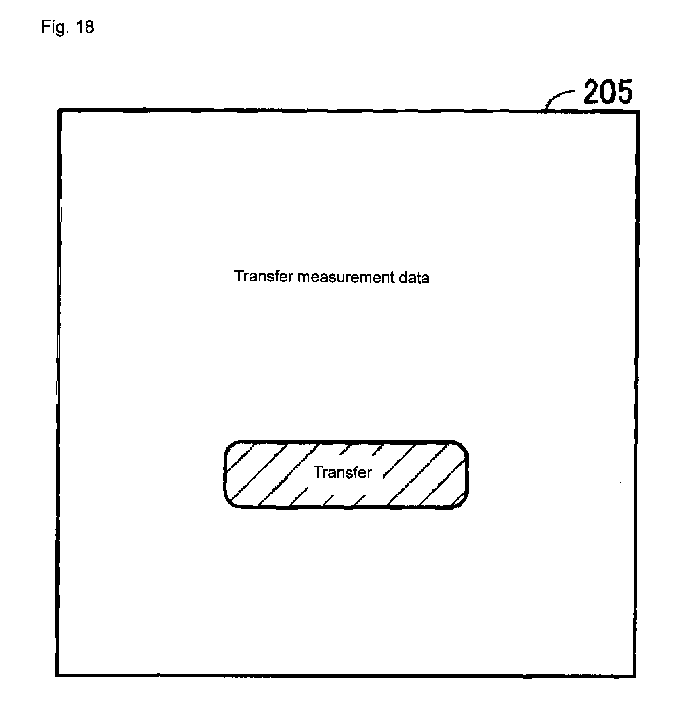

A body composition display system according to a second embodiment of the present invention will now be described using Figs. 17 and 18.

In the first embodiment, the specification program is selected according to the attribute or the usage purpose of the user. In the present embodiment, however, the specification program is selected according to the measurement results of two or more types of body compositions.

The second embodiment differs from the first embodiment in the specification program updating process. The configuration of the body composition display system and the configuration of each device in the second embodiment are the same as the first embodiment. Therefore, the difference with the first embodiment will be described citing the reference numerals used in Figs. 1 to 7.

In the present embodiment, the specification program is selected according to the measurement results of four types of body compositions in the body composition measuring instrument 100. The attribute correspondence table 81 and the usage purpose correspondence table 82 as shown in Fig. 8 thus may not be contained in the hard disc 306 of the server 300.

(Regarding specification program updating process)

Fig. 17 is a flowchart showing the specification program updating process executed by the body composition display system 1000 according to the second embodiment of the present invention.

With reference to Fig. 17, the communication terminal 200 accesses a home page provided by the server 300 based on instruction from the user (step S202). When a retrieval instruction of the measurement data is input by the user (step S204), the communication terminal 200 urges measurement to the user (step S206). Specifically, for example, a message "Measure body composition" is displayed on the display section 205.

The body composition measuring process is executed in the body composition measuring instrument 100 (step S208). The sub-routine of the body composition measuring process may be the same as the routine shown in Fig. 15 in the first embodiment. Thus, the detailed description will not be repeated herein.

After the body composition measuring process is terminated, the body composition measuring instrument 100 outputs the body information of the user and the measurement data to the communication terminal 200 (step S210). Specifically, the control section 12 of the body composition measuring instrument 100 reads out the age data, the sex data, and the height data stored in the body information storage region 41 corresponding to the personal number specified in the body composition measuring process in step S208, and the most recent measurement data (weight, body fat percentage, skeletal muscle percentage, visceral fat, basal metabolism) stored in the measurement result storage region 42, and outputs the read data to the communication terminal 200 by the data input/output section 18. In step S21 0, the body composition measuring instrument 100 also outputs information on the personal number to the communication terminal 200.

The communication terminal 200 receives the body information, the measurement data, and the personal number information through the input/output I/F 209 (step S212). A screen as shown in Fig. 18 is then displayed on the display section 205. With reference to Fig. 18, a message "transfer measurement data" and a button for instructing transfer are displayed on the display section 205.

When the user inputs the transfer instruction of the measurement data with such a screen being displayed (step S214), the communication terminal 200 transfers the body information and the measurement data received in step S212 to the server 300 (step S216). The personal number information received in step S212 is temporarily recorded in the RAM 203.

The server 300 receives the body information and the measurement data from the communication terminal 200 (step S218). The CPU 301 of the server 300 analyzes the received measurement data (step S220), and selects the specification program based on the analysis result (step S222). The detailed description of steps S220 and S222 will be hereinafter described.

The server 300 transmits information on the specification program selected in step S222 to the communication terminal 200 (step S224).

The communication terminal 200 displays the information of the specification program transmitted from the server 300 on the display section 205 (step S226). When instruction to download is input by the user (YES in step S228), the server 300 transmits the data of the specification program selected in step S222 to the communication terminal 200 via the communication I/F 307 (step S230). The communication terminal 200 receive the data of the program transmitted by the server 300 via the communication I/F 207, and transfers the same to the body composition measuring instrument 100 via the input/output I/F 209 (step S232). In this case, the communication terminal 200 also transmits the information on the personal number temporarily recorded in the RAM 203 to the body composition measuring instrument 100.

The data input/output section 18 of the body composition measuring instrument 100 receives data of the specification program and the information on the personal number (step S234). The control section 12 of the body composition measuring instrument 100 then updates the specification program corresponding to the relevant personal number (step S236).

The processes of steps S224 to S236 are similar to the processes of steps S18 to S30 of Fig. 10, and thus the detailed description will not be repeated.

If instruction to download is not input by the user in step S228, that is, if end is selected (NO in step S228), the specification program updating process is terminated.

In step S210, information for identifying the current (original) specification program (specification program stored in the specification program storage region 43) is also transmitted to the communication terminal 200, which may be transmitted from the communication terminal 200 to the server 300 in step S216. Information on whether or not the specification program selected in step S222 is the same as current specification program may then be displayed in the communication terminal 200.

In the present embodiment, the analyzing process of step S220 is performed based on the most recent measurement data, but is not limited thereto, and may be performed based on an average value of the past measurement data.

Specific processes in steps S220 and S222 will now be described.


(Regarding specific example of analyzing process of measurement data/selecting process of specification program)

In the present embodiment, determination on whether or not one or more specific body composition of the user satisfies the condition predefined is made as the analyzing process of the measurement data. Alternatively, determination (search) on which condition of the plurality of conditions predefined one or more specific body composition of the user satisfies is made. When making such determination, the conditions preferably differ according to the attribute of the user. Here, "attribute" may be the same as the attribute (age and sex) used in the first embodiment, or may include height.

As for the selecting process of the specification program, the specification program previously corresponded to the respective determination result is selected based on the result of the determination described above.

For instance, it is assumed that a predetermined standard value correspondence table (not shown) corresponded with the standard value (average value) of the visceral fat for every attribute is stored in the hard disc 306 of the server 300. In this case, the CPU 301 reads out the standard value (average value of the visceral fat of the same group as the attribute of the user) of the visceral fat corresponding to the attribute of the user from the standard value correspondence table in step S220. Whether or not the value of the visceral fat (specific body composition) of the user is greater than the read standard value is determined.

Thereafter, in step S222, the CPU 301 selects the specification program based on the determination result of whether or not the value of the visceral fat of the user satisfies a predetermined condition (greater than standard value). For instance, if the determination result of step S220 is that the predetermined condition is satisfied (i.e., if determined that the value of the visceral fat of the user is greater than the standard value), the specification program having the visceral fat as the main target of health management (e.g., correspond to usage purpose of the B program: lose weight to improve lifestyle-related diseases) is selected. If the determination result of step S220 is that the predetermined condition is not satisfied (i.e., if determined that the value of the visceral fat of the user is smaller than or equal to the standard value), and the user is a male, the specification program having the skeletal muscle percentage as the main target of health management (e.g., correspond to usage purpose of the C program: increase body strength) is selected. Alternatively, if the determination result of step S220 is that the predetermined condition is not satisfied (i.e., if determined that the value of the visceral fat of the user is smaller than or equal to the standard value), and the user is a female, the specification program having the body fat percentage as the main target of health management (e.g., correspond to usage purpose of the E program: lose weight for beatification) is selected.

Alternatively, in step S220, the CPU 301 may calculate the BMI of the user based on the height and the weight, which are body information of the user, and whether or not the predetermined condition is satisfied may be determined based on the value of the BMI and the value of the body fat percentage of the user. BMI is one type of body composition, and thus may be calculated by the body composition calculating unit 112 of the body composition measuring instrument 100 and included in one of the measurement data. Whether or not the BMI of the user is greater than or equal to 25, and the body fat percentage of the user is greater than or equal to 25% for male and 35% for female are determined.

Thereafter, in step S222, the CPU 301 selects the specification program based on the determination result on whether or not the BMI of the user and the value of the body fat percentage satisfy the predetermined condition. For instance, if the determination result in step S220 is that the predetermined condition is satisfied (i.e., if determined that BMI of the user is greater than or equal to 25, and the body fat percentage of the user is greater than or equal to 25% for male and 35% for female), the specification program having the visceral fat as the main target of health management (e.g., correspond to usage purpose of the B program: lose weight to improve lifestyle-related diseases) is selected. If the determination result of step S220 is that the predetermined condition is not satisfied (i.e., if determined that BMI of the user is lower than 25, or the body fat percentage of the user is lower than or equal to 25% for male and 35% for female), and the user is a male, the specification program having the skeletal muscle percentage as the main target of health management (e.g., correspond to usage purpose of the C program: increase body strength) is selected. Alternatively, if the determination result of step S220 is that the predetermined condition is not satisfied (i.e., if determined that BMI of the user is lower than 25, or the body fat percentage of the user is lower than or equal to 25% for male and 35% for female), and the user is a female, the specification program having the body fat percentage as the main target of health management (e.g., correspond to usage purpose of the E program: lose weight for beatification) is selected.

Thus, the correspondence table (not shown) in which the predetermined specification program is corresponded to the respective determination result merely needs to be stored in the hard disc 306 of the server 300. Various body compositions become the target of health management depending on the user, but if the value of the body composition of the user or the combination of the range of such value has a possibility of leading to lifestyle-related diseases, it is preferable that the visceral fat is preferentially made as the main target of health management. If the value of the body composition of the user or the combination of the range of such value has a possibility of relating to lack of exercise, it is preferable that the skeletal muscle percentage is preferentially made as the main target of health management. Similarly, if determined as slightly overweight, it is preferable that the body fat percentage is preferentially made as the main target of health management.

As described above, according to the present embodiment, the specification program suited for the user is selected based on the measurement data of the user. The selecting conditions of the specification program may include three conditions of attribute, usage purpose, and measurement result, and the user may specify one of such conditions. The server 300 may select the specification program according to the condition specified by the user.

[Third embodiment]

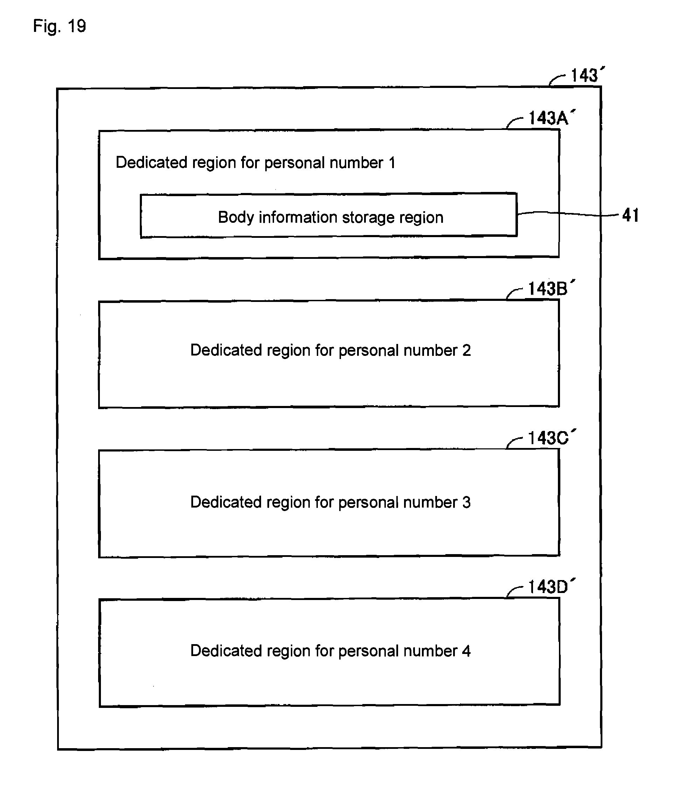

A body composition display system according to a third embodiment of the present invention will be described using Figs. 19 to 25.

In the first and second embodiments described above, the specification program is installed in the body composition measuring instrument, and the measurement result corresponding to the specification program is displayed in the body composition measuring instrument. In the present embodiment, on the other hand, the specification program is installed in the communication terminal, and the measurement result corresponding to the specification program is displayed in the communication terminal.

The configuration of the body composition display system and the basic configuration of each device in the third embodiment are similar to the first and second embodiments. Similar to the second embodiment, the specification program is selected according to the measurement result of two or more types of body composition in the third embodiment. Therefore, the difference with the second embodiment will be mainly described citing the reference numerals used in Figs. 1 to 3, 6, and 7.

Fig. 19 is a diagram showing a configuration example of a flash memory 143' of the body composition measuring instrument 100 according to the third embodiment of the present invention. The flash memory is denoted as 143' in the third embodiment to distinguish from the flash memory 143 in the second embodiment.

With reference to Fig. 19, similar to the second embodiment, the flash memory 143' includes a region 143'A for storing information about the user corresponding to personal number 1, a region 143'B for storing information about the user corresponding to personal number 2, a region 143'C for storing information about the user corresponding to personal number 3, and a region 143'D for storing information about the user corresponding to personal number 4.

The region 143'A includes only the body information storage region 41 for storing body information of the user corresponding to personal number 1. That is, the measurement result of the user corresponding to personal number 1, and the specification program of the user corresponding to personal number 1 are not stored in the region 143'. The regions 143'B to 143'D corresponding to other personal numbers also include the storage region similar to the region 143'A.

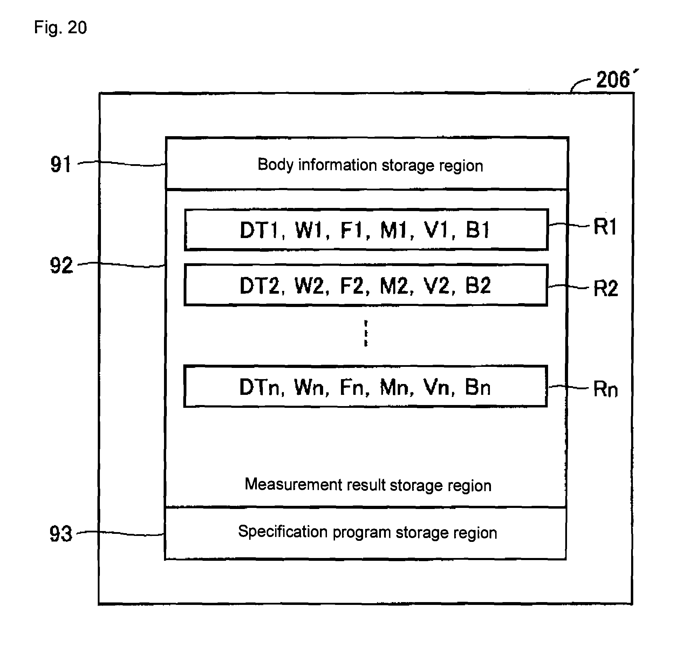

Fig. 20 is a diagram showing a configuration example of a flash memory 206' of the communication terminal 200 according to the third embodiment of the present invention. The flash memory is denoted as 206' in the third embodiment to distinguish from the flash memory 206 in the second embodiment.

The flash memory 206' includes a body information storage region 91 for storing body information of the user, a measurement result storage region 92 for storing the measurement result of the user, and a specification program storage region 93 for storing the specification program. The storage regions 91 to 93 store data corresponding to the storage regions 41 to 43 of the flush memory 143 in the body composition measuring instrument 100 of the second embodiment.

In the present embodiment, the memory button 16B and the personal buttons 16F, 16G, 16H, and 16I do not need to be included in the operating section 16.

The display control unit 122 of the body composition measuring instrument 100 does not perform the control of displaying the calculation result by the body composition calculating unit 121 on the display section 15, but the CPU 201 of the communication terminal 200 performs the control of displaying the measurement result (calculation result by the body composition calculating unit 121) in the body composition measuring instrument 100 on the display section 205.

In the present embodiment, the measurement result storing process, the specification program updating process, and the measurement result displaying process are performed independently as follows.

(Regarding measurement result storing process)

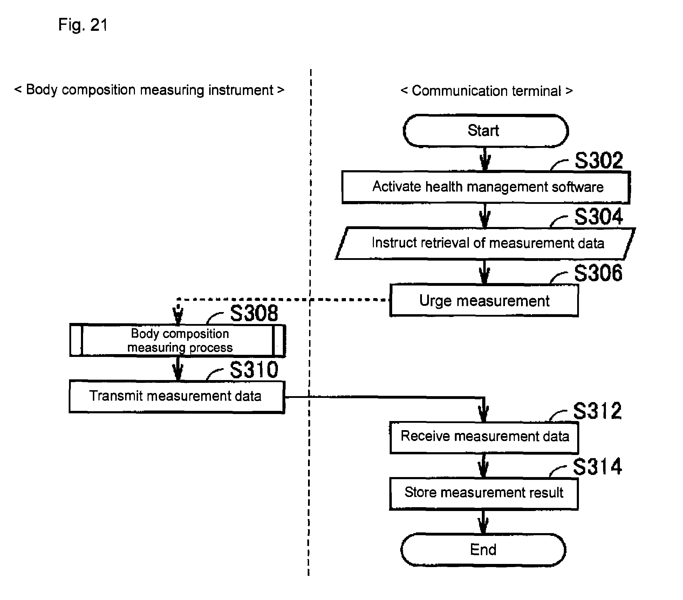

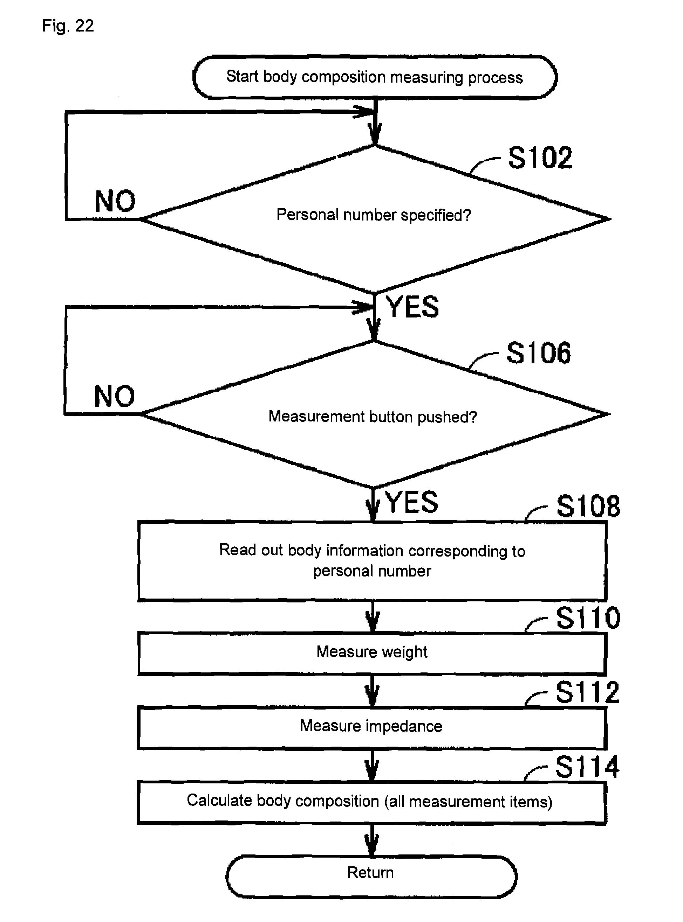

The measurement result storing process according to the present embodiment will be described using Figs. 21 and 22. Fig. 21 is a flowchart showing the measurement result storing process executed by the body composition measuring instrument 100 and the communication terminal 200 according to the third embodiment of the present invention.

With reference to Fig. 21, the communication terminal 200 activates software (hereinafter referred to as "health management software"), which enables the measurement result of the body composition to be displayed, installed in advance based on an instruction from the user (step S302). When the instruction to retrieve the measurement data is input by the user (step S304), the communication terminal 200 urges the measurement on the user (step S306). The process of step S306 may be the same as step S206 in Fig. 17 of the second embodiment.

In the body composition measuring instrument 100, the body composition measuring process is executed (step S308). Fig. 22 is a flowchart of a sub-routine showing the body composition measuring process according to the third embodiment of the present invention. The same step numbers are given to the processes similar to the flowchart shown in Fig. 15 in the first embodiment, and the description thereof will not be repeated.

With reference to Fig. 22, the processes of steps S102 to S114 are executed in the body composition calculating unit 121. Thereafter, the process returns to the main routine. That is, in the third embodiment, up to the calculation process of four types of body compositions are performed in the body composition measuring instrument 100, and the storing process and the displaying process of the measurement result are not performed.

After the measuring process of the body composition is terminated, the body composition measuring instrument 100 outputs the measurement data to the communication terminal 200 (step S310).

The communication terminal 200 receives the measurement data at the input/output I/F 209 (step S312). The CPU 201 of the communication terminal 200 stores the measurement data received at the input/output I/F 209 in the measurement result storage region 92 (step S314).

The measurement result storing process according to the third embodiment is thereby terminated.

(Regarding specification program updating process)

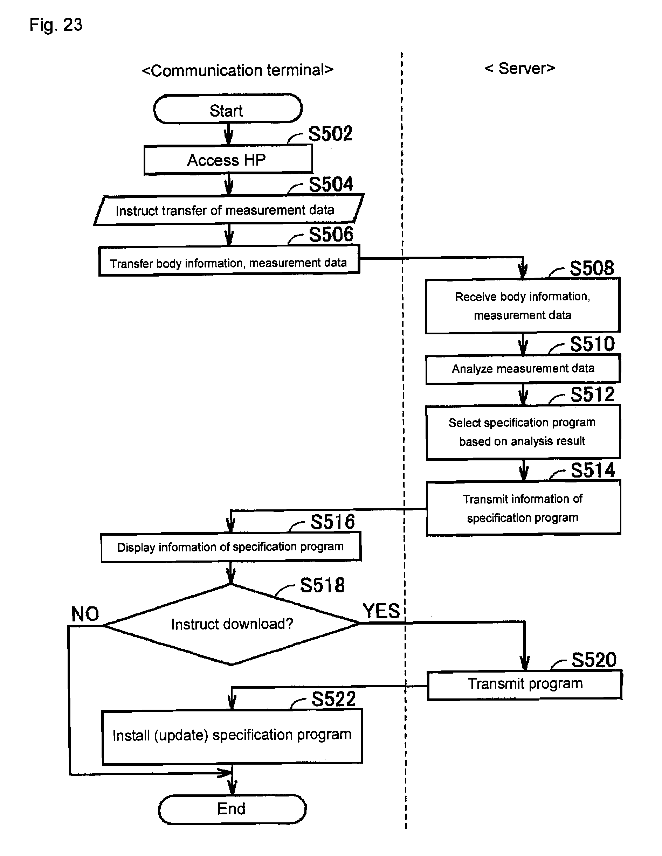

The specification program updating process according to the present embodiment will be described using Fig. 23.

Fig. 23 is a flowchart showing the specification program updating process executed by the communication terminal 200 and the server 300 according to the third embodiment.

With reference to Fig. 23, the communication terminal 200 accesses a home page provided by the server 300 based on instruction from the user (step S502). When the transfer instruction of the measurement data is input by the user (step S504), the communication terminal 200 transfers the body information and the most recent measurement data etc. stored in the flash memory 206' to the server 300 (step S506).

The server 300 receives the body information and the measurement data from the communication terminal 200 (step S508). The CPU 301 of the server 300 analyzes the received measurement data (step S51 0), and selects the specification program based on the analysis result (step S512). The specific processing procedures of steps S510 and S512 may be the same as the second embodiment.

The server 300 then transmits information of the selected specification program to the communication terminal 200 in step S514 (step S514).

The communication terminal 200 displays the information of the specification program transmitted from the server 300 on the display section 205 (step S516). When the instruction to download is input by the user (YES in step S518), the server 300 transmits the data of the specification program selected in step S512 to the communication terminal 200 via the communication I/F 307 (step S520). The communication terminal 200 receives the data of the program transmitted from the server 300 via the communication I/F 207, and installs (updates) the received specification program in the specification program storage region 93 of the flash memory 206' (step S522).

If the instruction to download is not input by the user that is, if end is selected in step S518 (NO in step S518), the specification program updating process is terminated.

(Regarding measurement result displaying process)

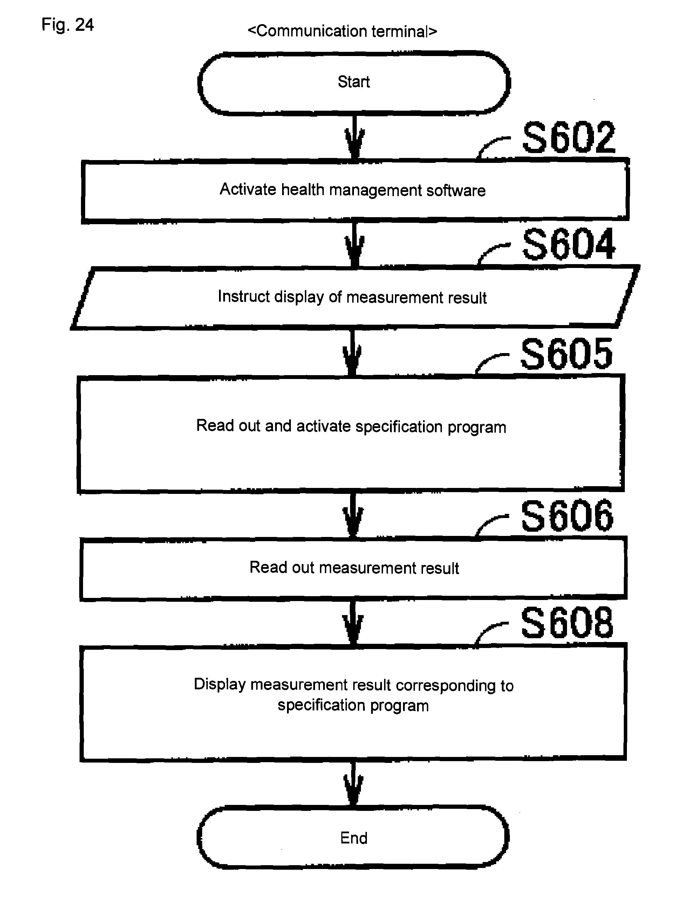

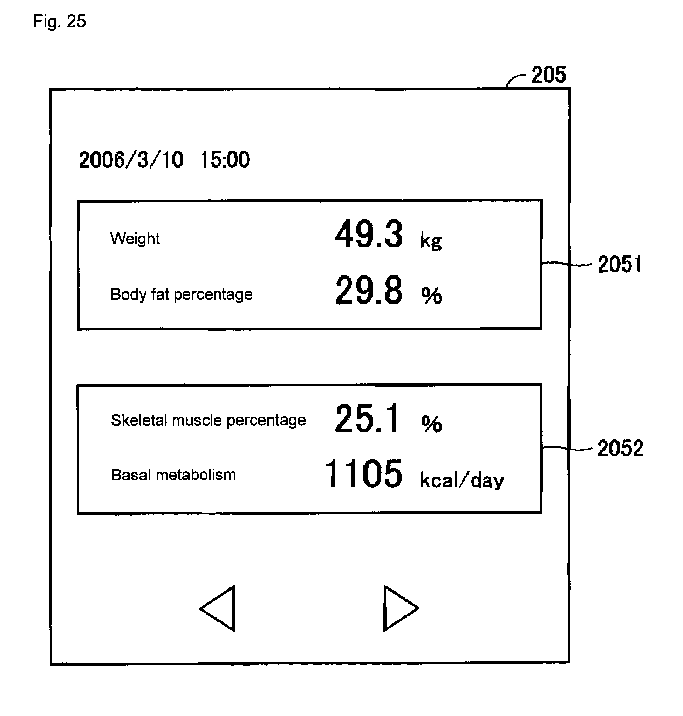

The measurement result displaying process according to the present embodiment will be described using Figs. 24 and 25. Fig. 24 is a flowchart showing the measurement result displaying process executed by the communication terminal 200 in the third embodiment.

With reference to Fig. 24, the health management software installed in advance is activated based on the instruction from the user (step S602). When the instruction to display the measurement result is then input by the user (step S604), the CPU 201 reads out and activates the specification program stored in the specification program storage region 93 (step S605).

When the specification program is activated, the CPU 201 reads out the measurement result stored in the measurement result storage region 92 (step S606), and executes the measurement result displaying process corresponding to the specification program (step S608). That is, the measurement results of the weight and the specific body composition are display according to the stored specification program of the read measurement results.

The measurement result displaying process of the third embodiment of the present invention is then terminated. Fig. 25 is a view showing one example of a screen displayed in step S608 of Fig. 24. In the figure, an example of a case where the E program is stored in the specification program storage region 93 as the specification program is shown.

With reference to Fig. 25, the weight and the body fat percentage are displayed in a first display region 2051, and the skeletal muscle percentage and the basal metabolism are displayed in a second display region 2052 in the display section 205. In this example, the measurement result for one round is displayed all at once. The past measurement results are sequentially displayed when a predetermined instruction is input via the operating section 204.

In the present embodiment, the user can browse through the past measurement results at the communication terminal 200 since the past measurement data are stored in the communication terminal 200. Therefore, if the communication terminal 200 is a portable terminal such as a portable telephone, the user can browse through the past measurement results irrespective of time and place.

In the present embodiment, the specification program having the type of body composition to be managed by the user as the displaying item is selected based on the measurement data of the user, similar to the second embodiment. The user thus can easily carry out his/her health management.

In the present embodiment as well, the specification program can be selected based on the attribute or the usage purpose of the user as in the first embodiment.

The specification program is selected based on one of the attribute, the usage purpose, and the measurement result of the user in the first to the third embodiments, but may be selected based on the combination thereof. Alternatively, without being limited to such conditions, the user can himself/herself select the displaying item (type of body composition) individually. Furthermore, the user can select the displaying order of the displaying item.

The embodiments disclosed herein are merely illustrative in all aspects and should not be construed as being restrictive. The scope of the invention is defined by the appended claims rather than by the description preceding them, and all changes that fall within meets and bounds of the claims, or equivalence of such meets and bounds are therefore intended to be embraced by the claims.

**Claims**

1. A body composition display system comprising:

   a body composition measuring instrument (100) including a measuring section for measuring two or more types of body compositions of a user; and
   a server (300) including
   a first storage section (81) for storing a plurality of predetermined display specification programs related to a specification of display of measurement results in the body composition measuring instrument,
   a selection control section (301) for performing a control to select one of the predetermined display specification programs based on at least one of attribute information related to an attribute of the user, purpose information related to a usage purpose of the user, and measurement data of the user input from a communication terminal (200) capable of exchanging data with the body composition measuring instrument, and
   a transmitting section (307) for transmitting the display specification program selected by the selection control section to the communication terminal via a communication network (400) as data of the selected program.

**2.** The body composition display system according to claim 1, wherein the body composition measuring instrument further includes,

an input/output section (18) for receiving input of the data of the selected program via the communication terminal,

a second storage section (143) for storing the input selected program,

a display control unit (122) for performing a control to display the measurement result of a specific body composition of the two or more types of body compositions based on the selected program stored in the second storage section, and

a display section (15) for displaying according to an output from the display control unit.

**3.** The body composition display system according to claim 1, wherein the communication terminal includes,

a receiving section (207) for receiving the data of the selected program via the communication network,

a second storage section (206) for storing the input selected program,

an input/output section (209) for receiving input of the measurement data of the two or more types of body compositions from the body composition measuring instrument,

a display control unit (201) for performing a control to display the measurement result of a specific body composition of the two or more types of body compositions based on the selected program stored in the second storage section, and

a display section (205) for displaying according to an output from the display control unit.

**4.** The body composition display system according to claim 2 or 3, wherein

the first storage section stores each of the predetermined display specification program in correspondence to a plurality of attributes; and

the selection control section selects the display specification program corresponded to the attribute of the user indicated by the attribute information when the attribute information is input from the communication terminal.

**5.** The body composition display system according to claim 2 or 3, wherein

the first storage section stores each of the predetermined display specification program in correspondence to a plurality of usage purposes; and

the selection control section selects the display specification program corresponded to the usage purpose of the user indicated by the purpose information when the purpose information is input from the communication terminal.

**6.** The body composition display system according to claim 2, wherein

the body composition measuring instrument further includes a third storage section (143) for storing the measurement data of the two or more types of body compositions measured by the measuring section;

the input/output section outputs the measurement data stored in the third storage section to the communication terminal; and

the selection control section includes,

an analyzing unit (301) for analyzing the measurement data when the measurement data is input from the communication terminal, and

a selecting unit (301) for selecting the display specification program based on the analysis result of the analyzing unit.

**7.** The body composition display system according to claim 3, wherein the communication terminal further includes a third storage section (206) for storing the input measurement data of the two or more types of body compositions; and

the selection control section includes,

an analyzing unit (301) for analyzing the measurement data when the measurement data is input from the communication terminal, and

a selecting unit (301) for selecting the display specification program based on the analysis result by the analyzing unit.

**8.** A body composition measuring instrument comprising:

measuring sections (E10, E20, 11, 12) for measuring two or more types of body compositions;

a storage section (143) for storing a first display specification program related to specification of display of measurement results by the measuring section;

a display control unit (122) for performing a control to display the measurement result of a specific body composition of the two or more types of body compositions based on the first display specification program;

a display section (15) for displaying according to an output from the display control unit;

an input section (18) for externally receiving input of data of a second display specification program; and

an updating section (12) for updating the first display specification program stored in the storage section to the second display specification program.

9. A communication terminal comprising:

an input/output section (209) for receiving input of measurement data of two or more types of body compositions from a body composition measuring instrument;

a storage section (206) for storing a first display specification program related to specification of display of the measurement result;

a display control unit (201) for performing a control to display the measurement result of a specific body composition of the two or more types of body compositions based on the first display specification program;

a display section (205) for displaying according to an output from the display control unit;

a receiving section (207) for externally receiving data of a second display specification program; and

an updating section (201) for updating the first display specification program stored in the storage section to the second display specification program.

10. A server (300) for performing a process of causing a communication terminal capable of exchanging data with a body composition measuring instrument for measuring two or more types of body compositions to display a measurement result in the body composition measuring instrument; the server comprising:

a storage section (306) for storing a plurality of predetermined display specification programs related to a specification of display of measurement results in the body composition measuring instrument;

a selection control section (301) for performing a control to select one of the predetermined display specification programs based on at least one of attribute information related to an attribute of the user, purpose information related to a usage purpose of the user, and measurement data of the user input from the communication terminal; and

a transmitting section (307) for transmitting the display specification program selected by the selection control section to the communication terminal as data of the program for displaying the measurement result of a specific body composition of the two or more types of body compositions on a display section of the communication terminal.

Fig. 1

1000

Body composition measuring instrument — 100

Communication terminal — 200

400

Server — 300

Fig. 2

Fig. 3

Fig. 4

┌─────────────────────────────────────────────── ⌐ 143
│
│   ┌──────────────────────────────────────── ⌐ 143A
│   │
│   │  Dedicated region for personal number 1
│   │
│   │   ┌──────────────────────────────────┐
│   │   │   Body information storage region │ ───── 41
│   │   ├──────────────────────────────────┤
│   │   │                                  │
│   │   │                                  │ ───── 42
│   │   │  Measurement result storage region│
│   │   │                                  │
│   │   ├──────────────────────────────────┤
│   │   │ Specification program storage region│ ──── 43
│   │   └──────────────────────────────────┘
│   └────────────────────────────────────────┘
│
│   ┌──────────────────────────────────────── ⌐ 143B
│   │   Dedicated region for personal number 2
│   └────────────────────────────────────────┘
│
│   ┌──────────────────────────────────────── ⌐ 143C
│   │   Dedicated region for personal number 3
│   └────────────────────────────────────────┘
│
│   ┌──────────────────────────────────────── ⌐ 143D
│   │   Dedicated region for personal number 4
│   └────────────────────────────────────────┘
│
└───────────────────────────────────────────────┘

Fig. 5

```
                                          ┌─ 42

  ┌─────────────────────────────────────────┐
  │  ┌─────────────────────────────────┐     │
  │  │   DT1, W1, F1, M1, V1, B1        │──── R1
  │  └─────────────────────────────────┘     │
  │  ┌─────────────────────────────────┐     │
  │  │   DT2, W2, F2, M2, V2, B2        │──── R2
  │  └─────────────────────────────────┘     │
  │                 ⋮                         │
  │  ┌─────────────────────────────────┐     │
  │  │   DTn, Wn, Fn, Mn, Vn, Bn        │──── Rn
  │  └─────────────────────────────────┘     │
  │                                          │
  └──────────────────────────────────────────┘
```

Fig. 6

Fig. 7

Fig. 8

| | Specification program | |
|---|---|---|
| Age range | Male | Female |
| 50 - 80 | A program | D program |
| 30 - 49 | B program | A program |
| 18 - 29 | C program | E program |

| Usage purpose | Specification program |
|---|---|
| Increase body strength | C program |
| Lose weight to improve lifestyle-related diseases | B program |
| Lose weight for beautification | E program |

Fig. 9

| Specification program | Displaying items | |
| --- | --- | --- |
| | First display screen | Second display screen |
| A program | Weight | Skeletal muscle percentage |
| | Body fat percentage | Visceral fat |
| B program | Weight | Body fat percentage |
| | Visceral fat | Skeletal muscle percentage |
| C program | Weight | Body fat percentage |
| | Skeletal muscle percentage | Basal metabolism |
| D program | Weight | Body fat percentage |
| | Visceral fat | Basal metabolism |
| E program | Weight | Skeletal muscle percentage |
| | Body fat percentage | Basal metabolism |

## Fig. 10

<Body composition measuring instrument >

< Communication terminal >

< Server >

Start

S2
Access HP

S4
Instruction to transmit screen of specification program menu

S6
Transmit screen of specification program menu

S8
Display screen of specification program menu

S10
Select condition and input personal number

S12
Transmit screen corresponding to selected condition

S13
Display screen corresponding to condition

S14
Select item

S16
Select specification program based on attribute, usage purpose correspondence table

S18
Transmit information of specification program

S20
Display information of specification program

S22
Instruct download?
NO    YES

S24
Transmit program

S26
Transfer program to body composition measuring instrument, Transmit personal number information

S28
Receive program, personal number information

S30
Update specification program corresponding to personal number

End

Fig. 11

205

Specification program menu

Attribute

Usage purpose

Select with

What is your personal number?    1

Fig. 12A

205

Attribute

What is your age?

18 to 29 years old

30 to 49 years old

50 to 80 years old

What is your sex?

Male

Female

Fig. 12B

~205

Usage purpose

Increase body strength

Lose weight to improve lifestyle-related diseases

Lose weight for beautification

Fig. 13

~ 205

Health management in E program
is recommended

Download

End

Fig. 14

15

Specification program of personal

number 1 is updated

Fig. 15

```
        ┌────────────────────────────────────────┐
        │  Start body composition measuring process │
        └────────────────────────────────────────┘
                          │
          ┌───────────────┤
          │               ▼                    ⌐S102
          │          ◇─────────────────────◇
       NO │          Personal number specified?
          │          ◇─────────────────────◇
          │               │ YES
          │               ▼                    ⌐S106
          │          ◇─────────────────────◇
       NO │          Measurement button pushed?
          │          ◇─────────────────────◇
          └───────────────│ YES
                          ▼                    ⌐S108
              ┌────────────────────────────┐
              │   Read out body information  │
              │ corresponding to personal number │
              └────────────────────────────┘
                          ▼                    ⌐S110
              ┌────────────────────────────┐
              │       Measure weight         │
              └────────────────────────────┘
                          ▼                    ⌐S112
              ┌────────────────────────────┐
              │      Measure impedance       │
              └────────────────────────────┘
                          ▼                    ⌐S114
              ┌────────────────────────────┐
              │ Calculate body composition (all measurement items) │
              └────────────────────────────┘
                          ▼                    ⌐S116
              ┌────────────────────────────┐
              │    Store measurement result  │
              └────────────────────────────┘
                          ▼                    ⌐S117
              ┌────────────────────────────┐
              │ Read out and activate specification program │
              │   corresponding to personal number │
              └────────────────────────────┘
                          ▼                    ⌐S118
              ┌────────────────────────────┐
              │ Measurement result displaying process │
              │ corresponding to specification program │
              └────────────────────────────┘
                          ▼
              ┌────────────────────────────┐
              │        End (/return)         │
              └────────────────────────────┘
```

Fig. 16A

15

Personal number 1

Weight        49.3 kg

Body fat percentage   29.8 %

Fig. 16B

Personal number 1

Skeletal muscle percentage 25.1 %

Basal metabolism 1105 kcal/day

15

## Fig. 17

< Body composition measuring instrument >   < Communication terminal >   < Server >

```
                                        ( Start )
                                           │ ⌐S202
                                     ┌──────────────┐
                                     │  Access HP   │
                                     └──────────────┘
                                           │ ⌐S204
                                    /Instruct retrieval of measurement/
                                    /          data                  /
                                           │ ⌐S206
                                     ┌──────────────┐
                                     │ Urge measurement │
                                     └──────────────┘
              │ ⌐S208
        ┌──────────────┐
        │ Body composition │
        │ measuring process │
        └──────────────┘
              │ ⌐S210
        ┌──────────────┐
        │ Transmit body │
        │ information,  │
        │ measurement data, │          │ ⌐S212
        │ personal number │       ┌──────────────┐
        │ information   │         │ Receive body information, │
        └──────────────┘          │ measurement data │
                                  └──────────────┘
                                           │ ⌐S214
                                    /Instruct transfer of measurement/
                                    /          data                  /
                                           │ ⌐S216
                                     ┌──────────────┐
                                     │ Transfer body information, │
                                     │ measurement data │
                                     └──────────────┘
                                                          │ ⌐S218
                                                    ┌──────────────┐
                                                    │ Receive body │
                                                    │ information, │
                                                    │ measurement data │
                                                    └──────────────┘
                                                          │ ⌐S220
                                                    ┌──────────────┐
                                                    │ Analyze measurement │
                                                    │ data │
                                                    └──────────────┘
                                                          │ ⌐S222
                                                    ┌──────────────┐
                                                    │ Select specification │
                                                    │ program based on │
                                                    │ analysis result │
                                                    └──────────────┘
                                                          │ ⌐S224
                                                    ┌──────────────┐
                                                    │ Transmit information of │
                                                    │ specification program │
                                           │ ⌐S226   └──────────────┘
                                     ┌──────────────┐
                                     │ Display information of specification │
                                     │ program │
                                     └──────────────┘
                                           │ ⌐S228
                              NO    ╱──────────────╲   YES
                              ◄─────◄ Instruct download? ►─────►
                                     ╲──────────────╱
                                                          │ ⌐S230
                                                    ┌──────────────┐
                                           │ ⌐S232   │ Transmit program │
                                     ┌──────────────┐ └──────────────┘
                                     │ Transfer program to body │
                        │ ⌐S234      │ composition measuring │
                  ┌──────────────┐   │ instrument, Transmit personal │
                  │ Receive program │ │ number information │
                  └──────────────┘   └──────────────┘
                        │ ⌐S236
                  ┌──────────────┐
                  │ Update specification │
                  │ program corresponding │
                  │ to personal number │
                  └──────────────┘
                        │
                  ( End )
```

Fig. 18

205

Transfer measurement data

Transfer

Fig. 19

143′

143A′

Dedicated region for personal number 1

Body information storage region ——41

143B′

Dedicated region for personal number 2

143C′

Dedicated region for personal number 3

143D′

Dedicated region for personal number 4

Fig. 20

206'

| Body information storage region | | 91 |
| DT1, W1, F1, M1, V1, B1 | R1 |
| DT2, W2, F2, M2, V2, B2 | R2 |
| ⋮ | |
| DTn, Wn, Fn, Mn, Vn, Bn | Rn |
| Measurement result storage region | |
| Specification program storage region | 93 |

92

Fig. 21

< Body composition measuring instrument >         < Communication terminal >

```
                                            ( Start )
                                                │
                                                ▼  S302
                                    ┌───────────────────────────────┐
                                    │ Activate health management software │
                                    └───────────────────────────────┘
                                                │
                                                ▼  S304
                                   ╱───────────────────────────────╱
                                   ╱ Instruct retrieval of measurement data ╱
                                   ╱───────────────────────────────╱
                                                │
                                                ▼  S306
                                    ┌───────────────────────────────┐
                            ┌ ─ ─ ─ │      Urge measurement           │
                            ▼  S308 └───────────────────────────────┘
                    ┌───────────────────┐
                    │  Body composition  │
                    │  measuring process │
                    └───────────────────┘
                            │  S310
                            ▼
                    ┌───────────────────┐
                    │ Transmit measurement data │──────────────┐
                    └───────────────────┘                      │
                                                               ▼  S312
                                                    ┌───────────────────────┐
                                                    │ Receive measurement data │
                                                    └───────────────────────┘
                                                               │
                                                               ▼  S314
                                                    ┌───────────────────────┐
                                                    │  Store measurement result │
                                                    └───────────────────────┘
                                                               │
                                                               ▼
                                                            ( End )
```

39

Fig. 22

```
        ( Start body composition measuring process )
                          │
        ┌─────────────────┤
        │                 ▼
        │         ╱────────────────────╲      S102
        │        ╱                      ╲
       NO ──────<   Personal number specified?  >
        │        ╲                      ╱
        │         ╲────────────────────╱
        │                 │ YES
        │                 ▼
        ┌─────────────────┤
        │                 ▼
        │         ╱────────────────────╲      S106
        │        ╱                      ╲
       NO ──────<  Measurement button pushed?  >
        │        ╲                      ╱
        │         ╲────────────────────╱
                          │ YES              S108
                          ▼
        ┌─────────────────────────────────────┐
        │  Read out body information           │
        │  corresponding to personal number    │
        └─────────────────────────────────────┘
                          │                   S110
                          ▼
        ┌─────────────────────────────────────┐
        │          Measure weight              │
        └─────────────────────────────────────┘
                          │                   S112
                          ▼
        ┌─────────────────────────────────────┐
        │        Measure impedance             │
        └─────────────────────────────────────┘
                          │                   S114
                          ▼
        ┌─────────────────────────────────────┐
        │ Calculate body composition           │
        │     (all measurement items)          │
        └─────────────────────────────────────┘
                          │
                          ▼
                  (      Return      )
```

Fig. 23

<Communication terminal>  < Server>

Start

S502
Access HP

S504
Instruct transfer of measurement data

S506
Transfer body information, measurement data

S508
Receive body information, measurement data

S510
Analyze measurement data

S512
Select specification program based on analysis result

S514
Transmit information of specification program

S516
Display information of specification program

S518
Instruct download?

NO        YES

S520
Transmit program

S522
Install (update) specification program

End

Fig. 24

<Communication terminal>

```
      ┌─────────────────────┐
      │        Start        │
      └─────────────────────┘
                 │
                 ▼                    ╭─ S602
┌───────────────────────────────────────┐
│    Activate health management software │
└───────────────────────────────────────┘
                 │
                 ▼                    ╭─ S604
 ╱─────────────────────────────────────╱
 ╱   Instruct display of measurement result ╱
╱─────────────────────────────────────╱
                 │
                 ▼                    ╭─ S605
┌───────────────────────────────────────┐
│                                         │
│   Read out and activate specification program │
│                                         │
└───────────────────────────────────────┘
                 │
                 ▼                    ╭─ S606
┌───────────────────────────────────────┐
│        Read out measurement result      │
└───────────────────────────────────────┘
                 │
                 ▼                    ╭─ S608
┌───────────────────────────────────────┐
│   Display measurement result corresponding to │
│           specification program         │
└───────────────────────────────────────┘
                 │
                 ▼
      ┌─────────────────────┐
      │         End         │
      └─────────────────────┘
```

Fig. 25

205

2006/3/10  15:00

Weight                          **49.3** kg

Body fat percentage             **29.8** %

2051

Skeletal muscle percentage      **25.1** %

Basal metabolism                **1105** kcal/day

2052

◁          ▷

## INTERNATIONAL SEARCH REPORT

International application No.

PCT/JP2007/058169

A. CLASSIFICATION OF SUBJECT MATTER
*A61B5/05*(2006.01)i

According to International Patent Classification (IPC) or to both national classification and IPC

B. FIELDS SEARCHED

Minimum documentation searched (classification system followed by classification symbols)
A61B5/05

Documentation searched other than minimum documentation to the extent that such documents are included in the fields searched
Jitsuyo Shinan Koho         1922-1996   Jitsuyo Shinan Toroku Koho   1996-2007
Kokai Jitsuyo Shinan Koho   1971-2007   Toroku Jitsuyo Shinan Koho   1994-2007

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 2005-532862 A (Biospase Co., Ltd.),<br>04 November, 2005 (04.11.05),<br>Par. Nos. [0018] to [0041]; Figs. 3 to 7<br>& WO 2004/006764 A1   & AU 2003-252419 A1<br>& KR 2004-0006331 A | 8,9<br>1-7,10 |
| A | JP 2004-255016 A (Matsushita Electric<br>Industrial Co., Ltd.),<br>16 September, 2004 (16.09.04),<br>Par. No. [0017]<br>(Family: none) | 1-10 |
| A | JP 2005-253610 A (Fukushima-Ken),<br>22 September, 2005 (22.09.05),<br>Par. Nos. [0017], [0041]<br>(Family: none) | 1-10 |

☐ Further documents are listed in the continuation of Box C.    ☐ See patent family annex.

*    Special categories of cited documents:
"A"  document defining the general state of the art which is not considered   to be of particular relevance
"E"  earlier application or patent but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

Date of the actual completion of the international search
25 June, 2007 (25.06.07)

Date of mailing of the international search report
10 July, 2007 (10.07.07)

Name and mailing address of the ISA/
Japanese Patent Office

Authorized officer

Facsimile No.

Telephone No.

Form PCT/ISA/210 (second sheet) (April 2005)

44

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005261488 A
- JP 2005052516 A
- JP 2003215122 A
- JP 2002083066 A